# EUROPÄISCHE PATENTSCHRIFT

(11) **EP 2 605 831 B1**
(45) Veröffentlichungstag und Bekanntmachung des Hinweises auf die Patenterteilung: **18.06.2014**
(21) Anmeldenummer: 11773679.3
(22) Anmeldetag: 11.10.2011
(51) Int. Cl.: A61P 31/00, A61P 11/10, A61P 11/12, A61K 36/185, A61K 36/28, A61K 36/25, A61K 36/11, A61K 36/53, A61K 36/235, A61K 36/35, A61K 36/57

(54) **VERFAHREN ZUR HERSTELLUNG ALKOHOLFREIER PFLANZENBASIERTER ZUSAMMENSETZUNGEN SOWIE AUF DIESE WEISE HERGESTELLTE ZUSAMMENSETZUNGEN UND DEREN VERWENDUNG**
PROCESS FOR THE MANUFACTURE OF ALCOHOL-FREE COMPOSITIONS ON THE BASIS OF PLANTS AND COMPOSITIONS OBTAINED BY SAID PROCESS AND THEIR USE.
PROCÉDÉ POUR LA PRODUCTION DES COMPOSITIONS SANS ALCOOL BASÉES SUR PLANTES ET COMPOSITIONS PRODUITES PAR CE PROCÉDÉ ET LEUR USAGE.

(30) Priorität: 10.12.2010 DE 102010054127
(43) Veröffentlichungstag der Anmeldung: 26.06.2013
(73) Patentinhaber: Maria Clementine Martin Klosterfrau Vertriebsgesellschaft mbH, 50670 Köln (DE)
(72) Erfinder: NAUERT, Christian, 41542 Dormagen (DE); GREVE, Harald, 40545 Düsseldorf (DE)
(74) Vertreter: Von Rohr Patentanwälte Partnerschaft mbB
(86) Internationale Anmeldenummer: PCT/EP2011/005076
(87) Internationale Veröffentlichungsnummer: WO 2012/076069

(56) Entgegenhaltungen:
- EP-A1- 0 702 957
- DE-A1- 19 756 677
- DATABASE TCM [Online] SIPO; 2. November 1994 (1994-11-02), Ma Chi: "Zixing Oral Liquid and process for preparation thereof / Oral liquid for treating carcinoma of lung and process for preparation thereof", XP002663568, Database accession no. CN-93104778-A & CN 1 094 293 A (MA CHI [CN]) 2. November 1994 (1994-11-02)
- DATABASE WPI Week 200632 Thomson Scientific, London, GB; AN 2006-299970 XP002663569, & CN 1 682 883 A (HAIWANG PHARM CO LTD) 19. Oktober 2005 (2005-10-19)
- SOHRABVANDI S. ET AL.: "Alcohol-free Beer: Methods of Production, Sensorial Defects, and Healthful Effects.", FOOD REVIEWS INTERNATIONAL, Bd. 26, Nr. 4, 4. Oktober 2010 (2010-10-04), Seiten 335-352, XP009154107, ISSN: 8755-9129, DOI: DOI:10.1080/87559129.2010.496022
- VOIGT R ET AL: "[Granulometric determination of the effect of polyvinylpyrrolidone on the dissolution rates of difficultyly soluble drugs (author's transl)].", DIE PHARMAZIE 1980 LNKD- PUBMED:7413707, Bd. 35, Nr. 5-6, 1980, Seiten 311-312, XP009154134, ISSN: 0031-7144

## Beschreibung

Die vorliegende Erfindung betrifft das Gebiet der Medizin, insbesondere das Gebiet der prophylaktischen und/oder therapeutischen Behandlung von Atemwegserkrankungen, Erkältungskrankheiten sowie grippalen Infekten und damit verbundenen Symptomen.

Insbesondere betrifft die vorliegende Erfindung ein Verfahren zur Herstellung einer zumindest im Wesentlichen alkoholfreien Zusammensetzung auf Basis mindestens eines Pflanzenextraktes mit sekretolytischer und/oder expektorationsfördernder Wirkung.

Des Weiteren betrifft die vorliegende Erfindung eine zumindest im Wesentlichen alkoholfreie Zusammensetzung auf Basis mindestens eines Pflanzenextraktes mit sekretolytischer und/oder expektorationsfördernder Wirkung, welche sich zur prophylaktischen und/oder therapeutischen Behandlung von Atemwegserkrankungen, Erkältungskrankheiten sowie grippalen Infekten und damit verbundenen Symptomen eignet.

Weiterhin betrifft die vorliegende Erfindung die Verwendung einer zumindest im Wesentlichen alkoholfreien Zusammensetzung auf Basis mindestens eines Pflanzenextraktes mit sekretolytischer und/oder expektorationsfördernder Wirkung zur prophylaktischen und/oder therapeutischen Behandlung von Atemwegserkrankungen, Erkältungskrankheiten sowie grippalen Infekten und damit verbundenen Symptomen bzw. zur Herstellung eines Arzneimittels zur Behandlung dieser Erkrankungen.

Der Begriff der Atemwegserkrankungen ist im Rahmen der vorliegenden Erfindung sehr weit zu verstehen und bezeichnet insbesondere sämtliche, insbesondere mit der Bildung von zähem Schleim und/oder Schleimhautentzündungen einhergehenden Erkrankungen der oberen wie unteren Atemwege, wobei hierunter insbesondere akute wie chronische entzündliche Krankheitszustände der Atemwege zu subsumieren sind. Beispiele für Atemwegserkrankungen der oberen Atemwege sind z. B. Erkältungen (z. B. Rhinitis) und grippale Infekte, während Beispiele für Atemwegserkrankungen der unteren Atemwege z. B. Bronchitis, Pneumonien oder dergleichen sind.

Der grippale Infekt, welcher im Rahmen der vorliegenden Erfindung synonym auch als Erkältung, Erkältungserkrankung bzw. Erkältungsinfekt bezeichnet wird, stellt insbesondere eine akute Infektionskrankheit insbesondere der Nasen-, Hals- und/oder Bronchialschleimhäute dar. Erkältungserkrankungen sind die häufigste Infektion des Menschen überhaupt: So erkranken Kleinkinder mehr als zehnmal im Jahr, während Erwachsene im Schnitt etwa zwei- bis dreimal im Jahr an einem grippalen Infekt erkranken.

Erreger für Erkältungserkrankungen sind im Allgemeinen Viren; zudem kann eine Erkältung zusätzlich auch von Bakterien verursacht werden. Was die als Krankheitsverursacher in Frage kommenden Viren anbelangt, so kommen diesbezüglich sehr verschiedene Viren aus unterschiedlichen Virusfamilien in Betracht, welche sich insbesondere durch eine besonders schnelle Vermehrung bzw. Replikation unter Ausbildung vieler unterschiedlicher Varianten auszeichnen. Beispielsweise können Viren aus der Familie der *Coronaviridae, Andenoviridae, Picornaviridae* und *Paramyxoviridae* Erkältungserkrankungen hervorrufen. Die Vielzahl verschiedener Viren und ihrer Subtypen erklärt, warum Menschen häufig an einer virusbedingten Erkältung erkranken können. Denn eine generelle Ausbildung einer Immunität ist aufgrund der hohen Anzahl und Variabilität der Viren nicht möglich. Die krankheitsverursachenden Viren werden sowohl als Tröpfcheninfektion durch die Luft als auch direkt oder indirekt durch Kontakt mit Erkrankten oder über kontaminierte Gegenstände per Kontaktinfektion oder Schmierinfektion übertragen.

Was den Verlauf der Erkältungserkrankung anbelangt, so kann dieser in zeitliche Phasen untergliedert werden. Die ersten Anzeichen eines grippalen Infektes sind meistens Halskratzen bis hin zu Halsschmerzen und Schluckbeschwerden, oft verbunden mit leichtem Frösteln. Als besonders typisches Erkältungssymptom tritt sehr oft zur gleichen Zeit eine Entzündung der Nasenschleimhäute auf, die auch Schnupfen (Rhinitis) genannt wird. Fast immer treten über einen Zeitraum von vier bis fünf Tagen Kopf- und Gliederschmerzen auf, und die Erkrankten fühlen sich häufig matt und abgeschlagen oder entwickeln sogar Fieber. Im Zuge von Erkältungen bzw. grippalen Infekten entwickelt sich meistens zusätzlich ein Reizhusten. Mit fortschreitender Infektion kommt es zur Bildung von zähflüssigem Schleim, welcher im Laufe der Genesung in Form von Auswurf durch unter Umständen schmerzhaftes Abhusten aus dem Körper entfernt wird.

Auch Atemwegserkrankungen der unteren Atemwege, wie z. B. Bronchitis, Asthma bronchiale, Pneumonien etc., sind häufig mit der Bildung von zähflüssigem Schleim sowie Schleimhautentzündungen und einem unangenehmen Husten verbunden.

Bei der Bronchitis handelt es sich um eine akute oder chronische Entzündung der Bronchien, welche im Krankheitsverlauf mit einer nachfolgenden Erkrankung der Bronchiolen verbunden sein kann. Die akute Bronchitis tritt meist in Verbindung mit Viruserkrankungen, wie z. B. Rhinitis, als sogenannte bakterielle Superinfektion auf. Ähnlich wie Atemwegserkrankungen der oberen Atemwege geht auch die Bronchitis im Allgemeinen mit Husten, Auswurf, einer leichten bis mittelschweren Temperaturerhöhung oder sogar Fieber, Thoraxschmerzen, zähem Sputum sowie Rasselgeräuschen beim Atmen einher.

Die bestehenden Therapieansätze und Therapiekonzepte zur Linderung der mit Atemwegserkrankungen einhergehenden Symptome sind im Allgemeinen unzureichend, da sie vielfach zu Unverträglichkeiten führen oder nicht ausreichend effektiv sind.

Die im Allgemeinen eingesetzten medikamentösen Therapien basieren häufig auf synthetischen Wirkstoffen, welche infolge ihrer chemischen Aktivität dauerhaften Schaden im Körper anrichten können. Weiterhin sind synthetische Mittel aufgrund ihrer komplizierten und langen Entwicklung und Herstellungsverfahren häufig deutlich kostenintensiver als naturbasierte Wirkstoffe bzw. Phytopharmaka.

Jedoch gehen auch mit pflanzlichen Wirkstoffen bzw. Phytopharmaka zahlreiche Probleme einher, da insbesondere zur Herstellung flüssiger Dosierungsformen die Wirkstoffe oftmals in Form von alkoholischen Extrakten mit relativ hohem Alkoholgehalt vorliegen. Eine effiziente Extraktion der medizinisch relevanten Inhaltsstoffe ist meist nämlich aufgrund der chemischen Eigenschaften der Wirk- bzw. Inhaltsstoffe nur unter Verwendung alkoholischer Extraktionsmittel möglich. Die Verabreichung alkoholischer Medikamente mit hohem Alkoholgehalt stellt in zahlreichen Fällen jedoch ein ernsthaftes Problem dar. Insbesondere für Säuglinge, Kleinkinder oder Kinder im Schulalter muss infolge des hohen Alkoholgehalts häufig auf synthetische und somit weniger verträgliche Medikationen zurückgegriffen werden. Weiterhin ist aufgrund der Rückfallgefahr die Einnahme alkoholhaltiger Medikamente mit hohem Alkoholgehalt für Alkoholiker nicht möglich. Auch Schwangerschaften oder Stillzeiten stellen Kontraindikationen zur oralen Anwendung bzw. Einnahme von alkoholhaltigen Medikamenten mit hohem Alkoholgehalt dar. Weiterhin kann die Einnahme alkoholischer Medikamente mit hohem Alkoholgehalt das Bewusstsein derart beeinträchtigen, dass das sichere Bedienen von Maschinen oder die Verkehrstauglichkeit nicht mehr gegeben sind.

Zwar existieren inzwischen durchaus Darreichungsformen, welche nur wenig oder sogar gar keinen Ethanol enthalten, aber dies nur aufgrund der Verwendung anderer, ebenfalls alkoholischer Extraktionsmittel, z. B. auf Glykol-Basis, wie Glycerin oder dergleichen.

Somit besteht ein Bedarf an Arzneimitteln oder pharmazeutischen Zusammensetzungen, insbesondere auf pflanzlicher Basis und bevorzugt mit sekretolytischer und/oder expektorationsfördernder Wirkung, welche weder Ethanol noch andere alkoholische Lösungsmittel aufweisen.

Es hat auch nicht an Versuchen gemangelt, alkoholfreie Medikationen auf Basis von alkoholischen Pflanzenflüssigextrakten herzustellen, indem die flüchtigen zur Gewinnung der medizinisch wertvollen Inhalts- bzw. Wirkstoffe notwendigen Extraktionsmittel, wie beispielsweise Ethanol, durch Vakuumtrocknung, Gefriertrocknung oder ähnliche Verfahren entfernt werden. Zwar können oftmals die alkoholischen Bestandteile erfolgreich entfernt werden, allerdings werden zum Teil auch die medizinisch wirksamen Inhalts- bzw. Wirkstoffe den Extrakten entzogen, wodurch diese zu therapeutischen Zwecken nicht immer uneingeschränkt verwendbar sind.

Auch andere Verfahren zur Entfernung der Auszugsmittel bzw. des Alkohols aus pflanzlichen Extrakten (wie z. B. Dialyseverfahren, destillative Verfahren, Evaporationsverfahren, Filtrationsverfahren, Lyophilisationsverfahren etc.) konnten sich bislang aufgrund der mangelnden Effizienz sowie der Unwirtschaftlichkeit dieser Verfahren nicht durchsetzen.

Die CN 1 094 293 A betrifft oral applizierbare Zusammensetzungen zur Behandlung von Lungenkarzinomen. Zum Erhalt derartiger Zusammensetzungen wird zunächst trockene Arnebia-Wurzel mit Ethanol extrahiert und aufkonzentriert. Anschließend erfolgt ein Wässerungsschritt in kaltem Ether mit darauffolgender Filtration und Destillation. Durch Trocknung wird schließlich der Wirkstoff Naphthoquinon-Pigment bereitgestellt, welches wiederum in Ethanol und Polyvinylpyrrolidon gelöst und gemischt wird. Anschließend wird die Ethanol-Komponente entfernt. Zur Bereitstellung eines Pulvers wird der aus den vorangegangenen Verfahrensschritten resultierende Feststoff auf Basis des Naphthoquinon-Pigments und Polyvinylpyrrolidon gemahlen. Zur Herstellung der oral applizierbaren Flüssigkeit wird das Pulver in heißem Wasser gelöst; zusätzlich werden Ginseng-Wurzel, Saponin und Inulin sowie darüber hinaus Ethylparaben- sowie Polypropylenglykol-Lösungen hinzugefügt.

Die EP 0 702 957 A1 betrifft peroral applizierbare Johanniskrautextrakte sowie ein Verfahren zu deren Herstellung. Dabei wird Johanniskrautfluidextrakten zwingend Polyvinylpyrrolidon zugegeben, und die Fluidphase wird anschließend bei erhöhter Temperatur bzw. unter Vakuum bis zur Trockne eingeengt. Es soll eine verbesserte Freisetzung der Dianthrone gegenüber Standardpräparaten resultieren.

Die DE 197 56 677 A1 betrifft peroral applizierbare Arzneipflanzentrockenextrakte, wobei die nichtflüchtige Phase des Extraktes an einen alkohollöslichen oder mit Alkohol mischbaren, wasserlöslichen oder mit Wasser mischbaren, bei Raumtemperatur festen Träger in mikrodisperser Form bzw. in Form einer halbfesten oder festen Lösung gebunden ist.

Die CN 1 682 883 A betrifft feste Sarcandra-Dispersionen, welche Sarcandra-Extrakt als pharmazeutischen Wirkstoff sowie ein Trägermaterial aus der Gruppe von Polyvinylpyrrolidon, Polyethylenglykol, Cyclodextrin, grenzflächenaktiven Substanzen, Carbamid, organischen Säuren, Zuckern und deren Mischungen enthalten. Die feste Sarcandra-Dispersion wird durch Mahlprozesse, Sprühtrocknung, Schmelzprozesse oder Lösungsprozesse hergestellt. Das finale Produkt kann schließlich in Form von Kapseln, Tabletten oder Granulaten vorliegen.

Somit besteht immer noch ein großer Bedarf an gut verträglichen Arzneimitteln auf der Basis von pflanzlichen Extrakten, insbesondere auf pflanzlicher Basis und bevorzugt mit sekretolytischer und/oder expektorationsfördernder Wirkung, welche jedoch weder Ethanol noch sonstige alkoholische Lösungsmittel bzw. Extraktionsmittel aufweisen.

Daher liegt der vorliegenden Erfindung die Aufgabe zugrunde, ein Verfahren zur Herstellung eines Arzneimittels und ein nach diesem Verfahren erhältliches Arzneimittel als solches bereitzustellen, wobei sich das Arzneimittel zur Behandlung von Atemwegserkrankungen, Erkältungskrankheiten sowie grippalen Infekten und damit verbundenen Symptomen eignen soll und einerseits pflanzliche Inhaltsstoffe bzw. Phytopharmaka in medizinisch wirkungsvoller Menge enthält, aber andererseits einen derart geringen Alkoholgehalt bzw. zumindest im Wesentlichen keinen Alkohol aufweist, dass die zuvor geschilderten, im Zusammenhang mit dem Stand der Technik auftretenden Nachteile und Nebenwirkungen wenigstens teilweise vermieden oder aber wenigstens zumindest teilweise abgeschwächt werden sollen.

Zur Lösung des zuvor geschilderten Problems schlägt die vorliegende Erfindung - gemäß einem **ersten** Erfindungsaspekt - ein Verfahren zur Herstellung einer zumindest im Wesentlichen alkoholfreien Zusammensetzung auf Basis mindestens eines Pflanzenextraktes mit sekretolytischer und/oder expektorationsfördemder Wirkung gemäß Anspruch 1 vor; weitere vorteilhafte Ausgestaltungen dieses Erfindungsaspekts sind Gegenstand der diesbezüglichen Unteransprüche.

Weiterer Gegenstand der vorliegenden Erfindung - gemäß einem **zweiten** Erfindungsaspekt - ist eine zumindest im Wesentlichen alkoholfreie Zusammensetzung auf Basis mindestens eines Pflanzenextraktes mit sekretolytischer und/oder expektorationsfördernder Wirkung gemäß Anspruch 8 bzw. 9, welche sich insbesondere zur prophylaktischen und/oder therapeutischen Behandlung von Atemwegserkrankungen, Erkältungskrankheiten sowie grippalen Infekten und damit verbundenen Symptomen eignet; weitere vorteilhafte Ausgestaltungen dieses Erfindungsaspekts sind Gegenstand der diesbezüglichen Unteransprüche.

Wiederum weiterer Gegenstand der vorliegenden Erfindung - gemäß einem **dritten** Erfindungsaspekt - ist die Verwendung einer erfindungsgemäßen Zusammensetzung zur Herstellung eines Arzneimittels zur prophylaktischen und/oder therapeutischen Behandlung von Atemwegserkrankungen, Erkältungskrankheiten sowie grippalen Infekten und damit verbundenen Symptomen gemäß Anspruch 15.

Es versteht sich von selbst, dass besondere Ausgestaltungen und Ausführungsformen, welche nur im Zusammenhang mit einem Erfindungsaspekt beschrieben sind, auch in Bezug auf die anderen Erfindungsaspekte entsprechend gelten, ohne dass dies ausdrücklich beschrieben ist.

Bei allen nachstehend genannten relativen bzw. prozentualen, insbesondere gewichtsbezogenen Mengenangaben ist zu beachten, dass diese Angaben im Rahmen der erfindungsgemäßen Zusammensetzung vom Fachmann derart auszuwählen bzw. zu kombinieren sind, dass in der Summe - gegebenenfalls unter Einbeziehung weiterer Komponenten bzw. Inhaltsstoffe bzw. Zusatzstoffe bzw. Bestandteile, insbesondere wie nachfolgend definiert - stets 100 % bzw. 100 Gew.-% resultieren. Dies versteht sich für den Fachmann aber von selbst.

Im Übrigen gilt, dass der Fachmann anwendungsbezogen oder einzelfallbedingt von den nachfolgend ausgeführten Mengenangaben abweichen kann, ohne dass er den Rahmen der vorliegenden Erfindung verlässt.

Gegenstand der vorliegenden Erfindung - gemäß einem **ersten** Aspekt der vorliegenden Erfindung - ist somit ein Verfahren zur Herstellung einer zumindest im Wesentlichen alkoholfreien Zusammensetzung mit einem Alkoholgehalt, insbesondere einem Ethanolgehalt, von weniger als 2 Vol.-% auf Basis mindestens eines Pflanzenextraktes mit sekretolytischer und/oder expektorationsfördernder Wirkung, wobei das Verfahren die folgenden Verfahrensschritte umfasst:
(a) Bereitstellung mindestens eines alkoholischen Pflanzenextraktes mit sekretolytischer und/oder expektorationsfördernder Wirkung, wobei der mindestens eine Pflanzenextrakt mit sekretolytischer und/oder expektorationsfördernder Wirkung ausgewählt ist aus der Gruppe von Thymian-Extrakt, Primelwurzel-Extrakt, Efeu-Extrakt, Pelargonie-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte; nachfolgend
(b) Aufbringen und/oder Immobilisieren des alkoholischen Pflanzenextraktes mit sekretolytischer und/oder expektorationsfördernder Wirkung an einem festen wasserlöslichen Träger, wobei der Träger ausgewählt wird aus der Gruppe von Zuckern, Zuckeraustauschstoffen und Kohlenhydraten sowie deren Kombinationen oder Mischungen; dann
(c) Entfernen des Alkohols durch Trocknung; anschließend
(d) Solubilisieren des auf diese Weise erhaltenen, von Alkohol befreiten, den Pflanzenextrakt mit sekretolytischer und/oder expektorationsfördernder Wirkung enthaltenden Trägers in einem wässrigen Exzipienten zum Erhalt einer im Wesentlichen alkoholfreien Zusammensetzung mit einem Alkoholgehalt, insbesondere einem Ethanolgehalt, von weniger als 2 Vol.-% auf Basis mindestens eines Pflanzenextraktes mit sekretolytischer und/oder expektorationsfördernder Wirkung.

Überraschenderweise wurde im Rahmen der vorliegenden Erfindung von der Anmelderin gefunden, dass sich durch das erfindungsgemäße Verfahren, welches unter anderem die Verfahrensschritte des Aufbringens bzw. Immobilisierens der alkoholischen Pflanzen-Extrakte an einem wasserlöslichen Träger, der Entfernung sämtlicher Alkohole sowie der Solubilisierung des extrakthaltigen Trägers in einem wässrigen Exzipienten umfasst, eine Zusammensetzung auf Basis mindestens eines Pflanzenextraktes mit sekretolytischer und/oder expektorationsfördernder Wirkung herstellen lässt, welche zumindest im Wesentlichen alkoholfrei ist und zur Behandlung von Atemwegserkrankungen, Erkältungskrankheiten, grippalen Infekten und den damit verbundenen Symptomen eingesetzt werden kann.

Dies ist insbesondere vollkommen überraschend, da im Fall von alkoholischen Pflanzenextrakten mit sekretolytischer und/oder expektorationsfördernder Wirkung nach dem Stand der Technik eine Entfernung des Alkohols oder des alkoholischen Extraktionsmittels nicht bzw. nicht ohne Weiteres (insbesondere nicht unter Substanzverlust) möglich ist, weil die in diesen Extrakten enthaltenen Wirk- und Inhaltsstoffe mit sekretolytischer und/oder expektorationsfördernder Wirkung (z. B. aromatische Verbindungen, ätherische Öle etc.) im allgemeinen volatil sind und bei der Entfernung des Alkohols oder des alkoholischen Extraktionsmittels mitgerissen würden. Im Rahmen der vorliegenden Erfindung ist es aber unerwarteterweise gelungen, dieses Problem in effizienter Weise zu lösen.

Der Begriff "pharmazeutische Zusammensetzung" - synonym bisweilen auch als "pharmazeutische Zubereitung" bezeichnet - ist im Rahmen der vorliegenden Erfindung sehr weit zu verstehen und bezeichnet nicht nur Arzneimittel, Medikamente, Pharmaka oder sonstige Medizinprodukte, welche laut Arzneimittelgesetz zur Diagnostik bzw. als Therapeutika bestimmt sind, sondern darüber hinaus auch Nahrungsergänzungsmittel oder sonstige nicht unter das Arzneimittelgesetz fallende Produkte, Zubereitungen oder Zusammensetzungen.

Der Begriff "zumindest im Wesentlichen alkoholfrei" ist diesbezüglich dahingehend zu verstehen, dass gemäß der deutschen Arzneimittelwarenverordnung der Ethanolgehalt der pharmazeutischen Zusammensetzung weniger als 0,05 g pro maximaler Einzeldosis beträgt. Weiterhin berücksichtigt der Begriff "zumindest im Wesentlichen alkoholfrei" auch die gemäß dem Europäischen Arzneibuch vorgegebene maximale Konzentration von verbleibenden Lösungsmitteln der Klasse 3, welche Ethanol umfassen, wonach eine maximale Konzentration des Ethanols von 5 g/kg bzw. 5.000 ppm im finalen Produkt gestattet ist, ohne dass die pharmazeutische Zusammensetzung als alkoholhaltig deklariert werden muss. Die Einnahme von Arzneimitteln mit Alkohol- bzw. Ethanolgehalten dieser Größenordnung ist auch bei Vorliegen der genannten Kontraindikationen unbedenklich.

Der Begriff "zumindest im Wesentlichen alkoholfrei" bezieht sich jedoch nicht nur auf den Ethanolgehalt, sondern vielmehr auf sämtliche alkoholische Substanzen bzw. Extraktionsmittel, welche insbesondere dem Auszug der pflanzlichen Wirk- und Inhaltsstoffe dienen können. Somit sind auch organische Lösungsmittel auf Glykol-Basis, wie z. B. Glycerin oder Polyethylenglykol, mitumfasst.

Was die Begriffe der "Sekretolyse" und der "Expektoration" anbelangt, so ist diesbezüglich Folgendes auszuführen:
Die vorgenannten, im Rahmen der vorliegenden Erfindung zu therapierenden Atemwegserkrankungen gehen aufgrund der Bildung von Sekreten mit einer mehr oder weniger stark ausgeprägten Expektoration von Sputum oder Bronchialsekret einher, welcher bzw. welches insbesondere Leukozyten, Epithelzellen, Staubteilchen, Rauchpartikel, Mikroorganismen etc. enthält. Der Begriff der Expektoration bezeichnet dabei das Aushusten dieser Sekrete bzw. des Sputums aus dem Bronchialsystem; die Expektoration spielt für den Heilungsprozess eine zentrale Rolle, da auf diesem Wege ein Großteil der Krankheitserreger, wie z. B. Viren oder Bakterien, aus dem Körper befördert wird.

Um einen erleichterten Auswurf des Sputums zu ermöglichen, kann der Prozess der Sekretolyse, welcher die Auflösung des zähen Schleims bezeichnet, durch die Gabe von Expektorantien (d. h. auswurffördernden Mitteln) oder sekretolytischen Mitteln bzw. Sekretolytika (d. h. schleimlösenden Mitteln) unterstützt und somit beschleunigt werden. Als Expektorantien werden im Rahmen der vorliegenden Erfindung insbesondere solche auswurffördernden Mittel bezeichnet, welche entweder einen sekretolytischen (d. h. also die Verflüssigung des Bronchialsekrets bewirkenden) Effekt oder aber einen sekretomotorischen (d. h. einen verstärkten Abtransport des Bronchialschleims bewirkenden) Effekt aufweisen.

Für weitere diesbezügliche Informationen zu den Begriffen der "Sekretolyse" und der "Expektoration" kann insbesondere verwiesen werden auf Pschyrembel, Klinisches Wörterbuch, Nikol Verlagsgesellschaft mbH, Hamburg, 257. Auflage, Stichwörter: *"Expektoration", "Expektorantien", "Sputum"* und *"Sekretolytika",* sowie die dort jeweils referierte Literatur, deren jeweiliger Inhalt hiermit durch Bezugnahme eingeschlossen ist.

Da eine beschleunigte Expektoration und eine geförderte Sekretolyse die Krankheitsdauer maßgeblich verkürzen, zielen zahlreiche Therapieansätze zur Behandlung von Atemwegserkrankungen insbesondere auf eine Unterstützung der Sekretolyse bzw. Unterstützung der Expektoration ab. Dies beinhaltet nach dem Stand der Technik insbesondere die Applikation synthetischer Wirkstoffe zur Verflüssigung des Bronchialsekrets. Weiterhin existieren auch phytopharmazeutische Ansätze zur Beschleunigung der Expektoration auf Basis von Wirkstoffen aus Heilpflanzen bzw. entsprechenden Arzneidrogen, welche meist weitaus verträglicher als synthetische Wirkstoffe sind.

Im Rahmen der vorliegenden Erfindung wird der mindestens eine Pflanzenextrakt mit sekretolytischer und/oder expektorationsfördernder Wirkung aus der Gruppe von Thymian-Extrakt, Primelwurzel-Extrakt, Efeu-Extrakt, Pelargonie-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte ausgewählt. Erfindungsgemäß bevorzugt sind Thymian-Extrakt und/oder Primelwurzel-Extrakt, insbesondere eine Kombination oder Mischung von Thymian-Extrakt und Primelwurzel-Extrakt.

Was die erfindungsgemäß eingesetzten Extrakte mit sekretolytischer und/oder expektorationsfördernder Wirkung anbelangt, so ist diesbezüglich Folgendes auszuführen:
Beim Echten Thymian *(Thymus vulgaris)* handelt es sich um eine Pflanzenart aus der Gattung der Thymiane *(Thymus)* aus der Familie der Lippenblütler *(Lamiaceae).* Hinsichtlich der chemischen Zusammensetzung seiner ätherischen Öle bzw. der Extrakte wird der Echte Thymian in sechs Chemotypen eingeteilt. Abhängig von klimatischen und genetischen Bedingungen der Pflanzenart sind Thymol, Geraniol, Linalool, α-Terpineol, Trans-thuyanol-4-terpineol-4 oder Carvacrol Hauptbestandteile der ätherischen Öle bzw. der Thymian-Extrakte. Insbesondere der Thymian-Inhaltstoff Thymol zählt zu den stärksten antibakteriell und antiviral wirkenden Einzelkomponenten von ätherischen Ölen und wird daher zur Behandlung von Entzündungen der Mund- und Rachenschleimhäute, Mundgeruch sowie kleineren Wunden eingesetzt. Weiterhin besitzt Thymol krampflösende, sekretionsfördemde und schmerzlindernde Eigenschaften, was insbesondere hinsichtlich des Einsatzes zur Behandlung von Katarrhen der oberen Luftwege, Erkältungskrankheiten bzw. grippalen Infekten, Bronchitis und Keuchhusten von Relevanz ist. Zudem werden den Polymethoxyflavonen und Monoterpenen des Thymians ebenfalls eine krampflösende und hustenreizstillende Wirkung zugesprochen.

Primeln (*Primula*) wiederum sind eine Pflanzengattung aus der Familie der Primelgewächse (*Primulaceae*), welche aufgrund ihres hohen Saponingehalts, insbesondere ihres hohen Primulasäuregehalts, auch als Heilpflanzen verwendet werden. Weiterhin enthalten Primeln wertvolle Inhaltsstoffe des pflanzlichen Sekundärmetabolismus, wie etwa Flavonoide und Carotinoide, sowie Spuren von ätherischem Öl und Enzymen wie der Primverase. Tee auf Basis von Bestandteilen der Primeln wirkt als mildes Sekretolytikum bei Husten, Bronchitis oder Erkältungskrankheiten. Primelwurzeln werden besonders häufig aufgrund ihres hohen Saponingehalts zur Gewinnung von Extrakten eingesetzt. Die Extrakte werden schließlich als Phytotherapeutika zur Verflüssigung des Sputums bei Erkrankungen der Atemwege mit Hustenreiz in Form von Hustentee, -säften und -tropfen verwendet.

Beim Efeu - systematisch *Hedera helix -* handelt es sich um eine immer grüne Mauerranke, welche in ganz Europa sowie Westasien vorkommt und deren Blätter häufig zur Herstellung von Phytopharmaka Verwendung finden. Die Blätter enthalten 5 bis 8 Gew.-% bidesmosidische Saponine, insbesondere Oleanolsäure, Hederagenin und Bayogenin, als pharmazeutisch wertvolle Inhaltsstoffe. In trockenen Blättern können sich durch Hydrolyse zudem die stärker wirkenden monodesmosidischen Saponine bilden. Weiterhin liegen im Efeu verschiedene Phenolverbindungen, Polyacetylen, Falcarinol, Falcarinon und 11,12-Dehydrofalcarinol vor. Die stark expektorierenden Eigenschaften der Saponine des Efeus werden durch eine indirekte Reizung des *Nervus vagus* im Magen erklärt. Die Anwendung erfolgt meist unter Verwendung der Blattextrakte, insbesondere bei Husten, spastischer Bronchitis und chronischem Atemwegskatarrh.

Bei der Pelargonie - systematisch *Pelargonium sidoides -* handelt es sich um eine Pflanze, die aufgrund ihrer Inhaltsstoffe mit sekretolytischer Wirkung in der Pharmakologie Anwendung finden. Die Wirkung der Pflanze ist vor allem auf die enthaltenen Cumarine, wie beispielsweise Umckalin, Gerbstoffe und ätherisches Öl zurückzuführen. Die Wirkstoffe der Pelargonie sind insbesondere deshalb relevant, da sie sowohl Bakterien an der Vermehrung hindern als auch Viren abwehren. Dies ist dahingehend von besonderer Bedeutung, dass es sich bei den meisten Atemwegserkrankungen um Superinfektionen von Bakterien und Viren handelt. Weiterhin lösen sie den zähen Schleim, der bei Atemwegserkrankungen vom Körper gebildet wird. Die Anwendungsgebiete von pelargoniumhaltigen Zusammensetzungen liegen somit in akuten sowie chronischen Infekten der Atemwege, insbesondere Bronchitis und grippalen Infekten.

Auch der Holunder - systematisch *Sambucus nigra -* besitzt Wirkstoffe, welche die Expektoration fördern bzw. beschleunigen. Zu diesen Inhaltsstoffen gehören Glycoside, ätherisches Öl, Flavonoide, Cholin, Schleimstoffe, Gerbstoffe, Gerbsäure sowie Vitamin C. Zur Herstellung von Phytopharmaka mit Inhaltsstoffen des Holunders werden insbesondere die Blüten der Pflanze verwendet. Solche Phytopharmaka werden insbesondere bei akuten Krankheiten der Atemwege, wie Husten, Bronchitis, Erkältungskrankheiten, Nebenhöhlenentzündungen oder auch allgemein zur Stärkung des Immunsystems, verwendet, da sie nicht nur schleimlösend sondern auch entzündungshemmend, pilztötend sowie allgemein anregend wirken.

Eine weitere Pflanze, welche sekretolytische Inhaltsstoffe enthält, ist der Fenchel - systematisch *Foeniculum vulgare.* Hier werden zur Herstellung von Phytopharmaka meistens die Früchte und Wurzeln eingesetzt. Als Inhaltsstoffe enthält Fenchel insbesondere ätherische Öle, aber auch Kieselsäure, Mineralsalze, Stärke sowie die Vitamine A, B und C. Fenchel hat ebenfalls eine stark schleimlösende und auswurffördernde Wirkung auf die Atemwege und hemmt weiterhin das bakterielle Keimwachstum.

Auch der Huflattich - systematisch *Tussilago farfara -* ist für seine pharmakologische Wirksamkeit bekannt. Als wirksame Inhaltsstoffe enthält er unter anderem Polysaccharide, Schleimstoffe, Styrole, Bitterstoffe und Gerbstoffe. Wie die bereits vorgenannten Heilpflanzen wirken Huflattich bzw. dessen Inhaltsstoffe bei Hustenreiz beruhigend und insbesondere schleimlösend. Als arzneilich wirksamer Teil zur Herstellung von Phytopharmaka werden im Allgemeinen die Blätter verwendet.

Beim Anis - *Pimpinella anisum -* handelt es sich um eine Pflanze, welche ursprünglich aus Asien und den südöstlichen Mittelmeerländern stammt und ebenfalls zur Behandlung von Atemwegserkrankungen dient. Vor allem wegen der geschmacklichen Eigenschaften ist Anis zur Herstellung von oral applizierbaren Zusammensetzungen beliebt. Die pharmazeutisch wirksamen Inhaltsstoffe von Anis umfassen vor allem ätherisches Öl, Anethol, Isoanethol, Anisketon, Anissäure, Acetylcholin, Kampfer, Eugenol, Kaffeesäure, Myristicin, Salicylate, Thymol, Xanthotoxin und Vitamin C. Anis ist zudem häufig Bestandteil von Teemischungen, wird aber auch in Form von ätherischem Öl eingesetzt. Die Besonderheit und spezielle Wirksamkeit des ätherischen Öls von Anis liegt insbesondere darin, dass es nach der Einnahme über die Lunge ausgeschieden wird und somit durch die Lungenbläschen und Bronchien von Innen wirkt.

Der Tüpfelfarn - systematisch *Polypodium vulgare -* ist eine Heilpflanze, deren pharmakologische Wirkung bereits seit dem Mittelalter bekannt ist. Heute wird der Tüpfelfarn in erster Linie als Expektorans eingesetzt, da er sich insbesondere zur Behandlung von Bronchitis eignet. Als wirksame Inhaltsstoffe enthält die Pflanze in erster Linie Gerbstoffe, Weichharz, Schleim, Eiweiß, apfelsaures Calcium sowie etwa 8 Gew.-% fettes Öl sowie Glycyrrhizin. Zudem wird den Wirkstoffen des Tüpfelfarns eine partielle hämolytische Wirkung zugeschrieben.

Die vorliegende Erfindung ist mit zahlreichen Vorteilen, Verbesserungen und Besonderheiten verbunden, welche die vorliegende Erfindung gegenüber dem Stand der Technik auszeichnen und welche in nicht beschränkender Weise wie folgt zusammengefasst werden können:
Die vorliegende Erfindung stellt erstmals ein effizientes Verfahren zur Herstellung einer zumindest im Wesentlichen alkoholfreien Zusammensetzung auf Basis mindestens eines Pflanzenextraktes mit sekretolytischer und/oder expektorationsfördernder Wirkung zur Verfügung, wobei sich die resultierende Zusammensetzung insbesondere zur Behandlung von Atemwegserkrankungen, insbesondere Erkältungskrankheiten, grippalen Infekten oder Bronchialerkrankungen etc., eignet. Eine effiziente, ökonomische und selektive Entfernung von Extraktionsmitteln, insbesondere Alkohol, zur Herstellung von alkoholfreien flüssigen Zusammensetzungen auf Basis von pflanzlichen Extrakten war bislang nämlich nicht in dieser Weise möglich.

In besonderem Maße ist dabei hervorzuheben, dass die erfindungsgemäße Zusammensetzung aufgrund des erfindungsgemäßen Verfahrens nicht nur frei von Ethanol ist, sondern auch keine anderen alkoholischen Lösungs- und Extraktionsmittel, insbesondere keine auf Glykol-Basis, aufweist.

Somit wird erstmals eine insbesondere pharmazeutische Zusammensetzung auf Basis mindestens eines Pflanzenextraktes mit sekretolytischer und/oder expektorationsfördernder Wirkung bereitgestellt, welche einen derart geringen Alkoholgehalt aufweist bzw. zumindest im Wesentlichen keinen Alkohol enthält, dass auch Patienten mit Kontraindikationen in Bezug auf die Einnahme alkoholhaltiger Medikamente gefahrlos behandelt werden können und auf die Einnahme synthetischer und deutlich stärkere Nebenwirkungen aufweisender Arzneimittel verzichtet werden kann.

Zudem zeichnen sich die pflanzlichen Wirk- bzw. Inhaltsstoffe der vorgenannten Pflanzen bzw. Arzneidrogen, wie beispielsweise das Thymol im Fall des Thymians oder die Saponine aus Primelwurzeln, durch ihre gute Verträglichkeit sowie ihre Effizienz beim Einsatz zur Behandlung von mit Atemwegserkrankungen einhergehenden Symptomen aus, insbesondere bei der Förderung der Expektoration bzw. bei der Beschleunigung der Sekretolyse von mit Atemwegserkrankungen einhergehendem zähem Schleim bzw. Bronchialsekret. Signifikante Nebenwirkungen sind in Bezug auf die Inhaltsstoffe der vorgenannten Pflanzen bzw. deren Extrakten als solche nicht bekannt. Vielmehr ist bei den diesbezüglichen Extrakten des Standes der Technik der hohe Alkohol-Anteil bzw. der hohe Anteil alkoholischer Extraktionsmittel in flüssigen Dosierungsformen das größere Problem, welches aber im Rahmen der Erfindung erfolgreich überwunden wurde.

Auch ist die Gefahr von weiteren Nebenwirkungen in der erfindungsgemäßen Zusammensetzung weiter reduziert, weil sämtliche alkoholische Extraktionsmittel entfernt worden sind. In der Folge besteht die Zusammensetzung im Wesentlichen aus einer Kombination lediglich der Wirk- bzw. Inhaltsstoffe der Extrakte sowie Wasser und gegebenenfalls weiteren alkoholfreien Inhaltsstoffen (z. B. Zucker, Zuckeraustauschstoffen etc.).

Weiterhin werden die pflanzlichen Inhalts- und Wirkstoffe der vorgenannten Pflanzen bzw. Arzneidrogen, so zum Beispiel das Thymol des Thymians oder die Saponine der Primelwurzeln, durch das erfindungsgemäße Verfahren in ihren Konzentrationen zumindest im Wesentlichen nicht verändert und liegen in medizinisch erforderlicher Menge in der finalen Zusammensetzung vor, was bislang das zentrale Problem bei der Herstellung alkoholfreier Zusammensetzungen auf Basis dieser pflanzlichen Extrakte darstellt. Die Wirkstoffe bleiben jedoch nicht nur in ihrer Konzentration erhalten, sondern werden auch in chemischer Hinsicht nicht verändert.

Somit wird eine zumindest im Wesentlichen alkoholfreie Zusammensetzung auf Basis mindestens eines Pflanzenextraktes mit sekretolytischer und/oder expektorationsfördernder Wirkung zur Behandlung von Atemwegserkrankungen bereitgestellt, welche nicht nur im Falle des Vorliegens von Kontraindikationen hinsichtlich der Einnahme alkoholhaltiger Medikationen besser geeignet ist, sondern auch bei Patienten ohne Kontraindikationen eine mit alkoholhaltigen Zubereitungen vergleichbare Dosierung bzw. Gabe der Wirk- und Inhaltsstoffe sicherstellt.

Auch die Produktion der erfindungsgemäßen Zusammensetzung ist äußerst ökonomisch, da die Prozessführung äußerst gut zu handhaben ist und die eingesetzten Stoffe, insbesondere die pflanzlichen Extrakte, sehr kostengünstig gewonnen werden können.

Weiterhin zeichnet sich die Zusammensetzung nach Art der vorliegenden Erfindung durch ihre hervorragenden organoleptischen Eigenschaften aus, wie auch die Anwendungsbeobachtungen zeigen. So ist die Zusammensetzung hinsichtlich ihres Geschmacks, aber auch in Bezug auf ihre Konsistenz als äuβerst angenehm einzustufen. Dies ist insbesondere dahingehend relevant, dass die Zusammensetzung gerade für Säuglinge und Kinder vorgesehen ist, die häufig die orale Aufnahme von Zubereitungen mit unangenehmem Geschmack verweigern.

Was das erfindungsgemäße Verfahren zur Herstellung einer zumindest im Wesentlichen alkoholfreien Zusammensetzung, insbesondere einer pharmazeutischen Zusammensetzung bzw. pharmazeutischen Zubereitung, auf Basis mindestens eines Pflanzenextraktes mit sekretolytischer und/oder expektorationsfördernder Wirkung anbelangt, so gliedert sich dieses Verfahren - wie zuvor beschrieben - in mindestens vier Verfahrensschritte (a) bis (d).

Wie zuvor geschildert, erfolgt in Verfahrensschritt (a) die Bereitstellung mindestens eines alkoholischen Pflanzenextraktes der vorgenannten Art.

Die Bereitstellung des oder der alkoholischen Pflanzenextrakte der vorgenannten Art, beispielsweise auf Basis von Thymian und/oder Primelwurzel, ist dem Fachmann als solches bekannt und erfolgt mit dem Fachmann an sich geläufigen Methoden, so dass es diesbezüglich keiner weitergehenden Ausführungen bedarf.

Erfindungsgemäß wird dabei in Verfahrensschritt (a) der mindestens eine alkoholische Pflanzenextrakt der vorgenannten Art mit einem Droge/Extrakt-Verhältnis im Bereich von 1 : 10 bis 10 : 1, insbesondere im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, bereitgestellt.

Das Droge/Extrakt-Verhältnis (DEV) gibt an, aus welcher Menge an eingesetzter Droge, z. B. Thymian, Primelwurzel etc., welche Menge Extrakt gewonnen wurde. Ein Droge/Extrakt-Verhältnis von z. B. 6 : 1 bedeutet, dass aus sechs Tonnen Droge eine Tonne Extrakt gewonnen wurde. Das Droge/Extrakt-Verhältnis gibt also an, wie viele Teile einer Arzneidroge für die Herstellung des Extraktäquivalents benötigt wurden.

Die Verwendung von Extrakten mit streng definiertem Droge/Extrakt-Verhältnis ist dahingehend relevant, dass die Qualität des Extraktes einen entscheidenden Einfluss auf die Gesamtqualität der pharmazeutischen Zubereitung hat. Ziel ist dabei, den Extrakt hinsichtlich der Wirkstoffkonzentration zu standardisieren, so dass auch die finale pharmazeutische Zubereitung qualitativ und quantitativ mit konstant guten Werten bezüglich der Wirk- und Inhaltsstoffe bereitgestellt werden kann.

Das erfindungsgemäß ausgewählte Droge/Extrakt-Verhältnis gewährleistet insbesondere eine ausreichende Wirkstoffkonzentration, um zu einem pharmazeutisch bzw. medizinisch wirksamen Endprodukt zu gelangen. Andererseits lässt sich jedoch ein Extrakt mit diesem Droge/Extrakt-Verhältnis ökonomisch und gut handhabbar herstellen. Dabei sind dem Fachmann die zur Herstellung erforderlichen Parameter, wie die Wahl des Auszugsmittels sowie der Extraktionsbedingungen, als solche bekannt. Bevorzugte Auszugsmittel sind dabei insbesondere Ethanol, aber auch andersartige Auszugsmittel, wie beispielsweise Mischungen von Ammoniaklösungen und glykolbasierten Lösungsmitteln, wie Glycerin. Sowohl Mischextrakte, zum Beispiel Mischextrakt von Thymian und Primelwurzel, als auch Einzelextrakte der vorgenannten Pflanzen bzw. Arzneidrogen werden mittels der Auswahl des Auszugsmittels sowie der Extraktionsbedingungen auf das zuvor angegebene Droge/Extrakt-Verhältnis eingestellt.

Insbesondere ist es erfindungsgemäß vorgesehen, dass in Verfahrensschritt (a) der mindestens eine alkoholische Pflanzenextrakt mit einem Gehalt an Alkohol, insbesondere Ethanol, von mehr als 40 Vol.-% und/oder mehr als 40 Gew.-%, insbesondere mehr als 50 Vol.-% und/oder mehr als 50 Gew.-%, besonders bevorzugt mehr als 60 Vol.-% und/oder mehr als 60 Gew.-%, bereitgestellt wird.

Dabei ist es erfindungsgemäß üblicherweise vorgesehen, dass in Verfahrensschritt (a) der mindestens eine alkoholische Pflanzenextrakt mit einem Gehalt an Alkohol, insbesondere Ethanol, im Bereich von 40 Vol.-% bis 99 Vol.-% und/oder im Bereich von 40 Gew.-% bis 99 Gew.-%, insbesondere im Bereich von 50 Vol.-% bis 95 Vol.-% und/oder im Bereich von 50 Gew.-% bis 95 Gew.-%, besonders bevorzugt im Bereich von 60 Vol.-% bis 90 Vol.-% und/oder im Bereich von 60 Gew.-% bis 90 Gew.-%, bereitgestellt wird.

Der Alkoholgehalt in Extrakten von pflanzlichen Wirk- bzw. Inhaltsstoffen ist hinsichtlich der Effizienz der Extraktion von zentraler Bedeutung, da sich die pflanzlichen Wirk- bzw. Inhaltsstoffe, insbesondere apolare organische Verbindungen des pflanzlichen Sekundärmetabolismus, wie Carotinoide, oder phenolische Substanzen, wie Thymol, meist nur in organischen Lösungsmitteln, wie beispielsweise Ethanol oder Glycerin, jedoch nicht in wässrigen Lösungsmitteln effizient lösen. Sowohl Mischextrakte als auch Einzelextrakte, beispielsweise solche von Thymian und/oder Primelwurzeln, werden auf den definierten Alkoholgehalt eingestellt, um eine optimale Extraktion der Inhalts- bzw. Wirkstoffe sicherzustellen.

Bevorzugterweise erfolgt Verfahrensschritt (a) unter Bereitstellung einer Kombination von mindestens zwei alkoholischen Pflanzenextrakten, wobei bevorzugterweise jedoch beide Extrakte zunächst als Einzelextrakte bereitgestellt und erst nachfolgend gemischt bzw. kombiniert werden. Die Bereitstellung in Form von Einzelextrakten ist dahingehend vorteilhaft, dass sich durch die spezielle Wahl unterschiedlicher Auszugsmittel die Einzelextrakte jeweils mit höherer Effizienz herstellen lassen, als dies im Falle eines Mischextraktes der Fall ist.

Dies wird nachfolgend - rein beispielhaft - an einer Kombination von Thymian-Extrakt einerseits und Primelwurzel-Extrakt andererseits erläutert: So kann beispielsweise die Herstellung von Thymianflüssigextrakt besonders effizient unter Verwendung von 10%iger Ammoniaklösung, 85%igem Glycerin, 90%igem Ethanol sowie Wasser in einem Verhältnis von 1 : 20 : 70 : 109 als Auszugsmittel erfolgen. Die Wirkstoffe anderer Pflanzen hingegen stellen aufgrund ihrer anderen chemischen Eigenschaften andere Anforderungen an das Extraktionsmittel. So lassen sich beispielsweise die Saponine der Primelwurzeln zur Herstellung eines Primelwurzelflüssigextraktes wesentlich effizienter z. B. unter ausschließlicher Verwendung von 70%igem Ethanol als Auszugsmittels extrahieren. Wie das Beispiels der Thymianextrakt/Primelwurzelextrakt-Kombination zeigt, wäre im Falle eines Mischextraktes eine optimale Extraktion kaum möglich, da dazu sehr unterschiedliche Auszugsmittel benötigt werden.

Ein weiterer Vorteil der Kombination von Einzelextrakten gegenüber Mischextrakten (d. h. Extrakten, bei denen Bestandteile von zwei oder mehr Arzneidrogen gemeinsam extrahiert werden) ist die Tatsache, dass die Wirkstoffkonzentration bei der Kombination von Einzelextrakten höher ist, da die Extraktion jeweils für den jeweiligen Wirkstoff bzw. die jeweilige Pflanze (Arzneidroge) optimiert durchgeführt werden kann.

Im Rahmen des sich an Verfahrensschritt (a) anschließenden Verfahrensschritts (b) des erfindungsgemäßen Verfahrens erfolgt - wie zuvor geschildert - ein Aufbringen bzw. Immobilisieren des mindestens einen alkoholischen Pflanzenextraktes an einem festen wasserlöslichen Träger.

Hinsichtlich der insbesondere mit Verfahrensschritt (b) benutzten Bezeichnungen "Aufbringen" bzw. "Immobilisieren" ist anzumerken, dass diese synonym für weitere Begriffe mit artverwandter Bedeutung, wie beispielsweise "Fixierung" oder "Anbindung", verwendet werden, welche gleichermaßen die dauerhafte Anbindung des mindestens einen alkoholischen Pflanzenextraktes an dem festen wasserlöslichen Träger umfassen.

Weiterhin ist hinsichtlich der Formulierung "an einem wasserlöslichen Träger" zu erwähnen, dass hiermit sowohl die Fixierung auf dem Träger - vergleichbar mit einer oberflächlichen Beschichtung - als auch das Einziehen in das Trägermaterial (d. h. in das Innere des Trägermaterials) umfasst sind.

Insbesondere kann erfindungsgemäß in Verfahrensschritt (b) das Aufbringen und/oder Immobilisieren des mindestens einen alkoholischen Pflanzenextraktes an dem festen wasserlöslichen Träger mittels Granulierung, Agglomerierung, Pelletierung oder Coating, vorzugsweise mittels Granulierung, erfolgen.

Das Aufbringen bzw. Immobilisieren des oder der alkoholischen Extrakte mittels dieser Verfahren ist dem Fachmann als solches geläufig und bedarf an dieser Stelle keiner weiteren Ausführungen.

Gemäß einer besonders bevorzugten Ausführungsform des erfindungsgemäβen Verfahrens erfolgt in Verfahrensschritt (b) die Granulierung mittels Wirbelschichtgranulierung, Trockengranulierung oder Sprühgranulierung, vorzugsweise mittels Wirbelschichtgranulierung und/oder Sprühgranulierung.

Eine Granulierung mit Hilfe der zuvor genannten Granulierungsverfahren eignet sich insbesondere zur Immobilisierung bzw. zum Aufbringen der Extrakte an dem Träger, da sich auf diese Weise Parameter, wie beispielsweise Partikelgröße, Kompressibilität oder Schüttdichte, besonders gut einstellen lassen. Weiterhin handelt es sich hierbei um äußerst effiziente sowie leicht zu handhabende Verfahren, welche dem Fachmann als solche bekannt sind. Durch die Technik des Granulierens werden Teilchen erhalten, welche sich besonders gut im weiteren Herstellungsverfahren verarbeiten lassen.

Für weitergehende Einzelheiten zu Granulierverfahren, insbesondere Wirbelschichtverfahren (Wirbelbettverfahren), im Allgemeinen kann auf die allgemeine Fachliteratur verwiesen werden, beispielsweise auf Römpp Chemielexikon, Georg Thieme Verlag, Stuttgart/New York, 10. Auflage, Stichwörter: "Granulate", "Prillen" und "Wirbelschichtverfahren", sowie die dort jeweils referierte Literatur, deren jeweiliger Inhalt hiermit durch Bezugnahme eingeschlossen ist.

Erfindungsgemäß wird in Verfahrensschritt (b) ein partikulärer Träger eingesetzt, welcher ausgewählt wird aus der Gruppe von Zuckern, Zuckeraustauschstoffen und Kohlenhydraten sowie deren Kombination oder Mischungen, vorzugsweise aus Mono-, Di-, Tri- und Polysacchariden, besonders bevorzugt aus Disacchariden, ganz besonders bevorzugt aus Saccharose und/oder Saccharose-Derivaten.

Durch die partikuläre Größe des Trägers wird einerseits eine ausreichend groβe Fläche zur Anbringung der Extrakte bereitgestellt. Andererseits wird sichergestellt, dass die Effizienz des Verfahrens nicht durch schwer zu verarbeitende Pulver bzw. Stäube beeinträchtigt wird.

Der Begriff "Zucker" wird im Rahmen der vorliegenden Erfindung insbesondere für süßschmeckende Saccharide (z. B. Einfach- und Doppelzucker), insbesondere jedoch für den Doppelzucker Saccharose, welcher dem gewöhnlichen Haushaltszucker entspricht, verwendet. Zucker ist besonders gut als Träger zur Immobilisierung bzw. zum Auftragen der eingesetzten Flüssigextrakte geeignet, da er wasserlöslich ist und die Extrakte gut an ihm haften. Auch wird durch die Zuckerkörner aufgrund ihrer Größe einerseits eine ausreichende Fläche zur Auftragung bzw. Immobilisierung der Extrakte bereitgestellt; andererseits sind die Partikel aufgrund ihrer Größe staubfrei. Weiterhin verleiht er der finalen pharmazeutischen Zusammensetzung bereits nach dem Solubilisieren eine angenehme Süße, was das Erfordernis von weiteren Zusatzstoffen zur Geschmacksmodifizierung vermindert bzw. entbehrlich macht. Unter dem Begriff "Zuckeraustauschstoffe", wie er erfindungsgemäß verwendet wird, versteht man eine Sammelbezeichnung für Stoffe, welche anstelle von Saccharose einerseits zur Süßung der pharmazeutischen Zusammensetzung, andererseits insbesondere aber auch als Träger von Wirk- bzw. Inhaltsstoffen dienen können. Dabei eignen sie sich auch besonders gut zur Verarbeitung mittels Granulier-, Agglomerier-, oder Coating-Verfahren. Denn Zuckeraustauschstoffe werden technologisch wie Saccharose eingesetzt, d. h. sie besitzen einen "Körper" und einen physiologischen Brennwert (nutritive Zuckeraustauschstoffe). Der physiologische Vorteil der Zuckeraustauschstoffe im Vergleich zur Saccharose liegt in der insulinunabhängigen Metabolisierung (Diabetiker) und in der zum Teil verminderten kariogenen Wirkung. Für weitergehende Einzelheiten zu dem Begriff der Zuckeraustauschstoffe kann beispielsweise verwiesen werden auf Römpp Lexikon Chemie, Band 6, 10. Auflage, Georg Thieme Verlag, Stuttgart/New York, 1999, Seite 5098 bis 5100, Stichwort: "Zuckeraustauschstoffe", und auf Römpp Lexikon Lebensmittelchemie, 9. Auflage, Georg Thieme Verlag, Stuttgart/New York, Seite 955, Stichwort: "Zuckeraustauschstoffe", sowie die dort jeweils referierte Literatur, deren jeweiliger Inhalt hiermit durch Bezugnahme eingeschlossen ist.

Gemäß einer bevorzugten Ausführungsform erfolgt in Verfahrensschritt (b) das Aufbringen und/oder Immobilisieren des mindestens einen alkoholischen Pflanzenextraktes an dem Träger unter Verwendung mindestens eines weiteren Additivs, vorzugsweise mindestens eines Bindemittels und/oder Granulatbildners. Erfindungsgemäß handelt es sich bei dem Granulatbildner um einen pharmazeutisch verträglichen Hilfsstoff, bevorzugterweise um Polyvinylpyrrolidon. Der Granulatbildner - synonym auch als Granulierhilfsmittel, Granuliersubstanz etc. bezeichnet - dient gewissermaßen als Gerüstsubstanz bzw. sozusagen als "Klebstoff" für die zu granulierenden Inhaltsstoffe. Der erfindungsgemäß eingesetzte Granulatbildner sollte vorteilhafterweise wasserlöslich oder zumindest aber wasserdispergierbar, vorzugsweise wasserlöslich, ausgebildet sein, um einerseits bei der Granulierung als Lösung eingesprüht werden zu können und um sich andererseits zur Herstellung der finalen pharmazeutischen Zusammensetzung solubilisieren zu lassen. Um sicherzustellen, dass der Granulatbildner keine unerwünschten Nebenwirkungen bei medizinischer Anwendung hat, sollte es sich dabei um einen pharmazeutisch anerkannten Hilfsstoff, wie beispielsweise Polyvinylpyrrolidon, handeln.

Das mindestens eine Additiv, insbesondere der Granulatbildner, wird gemäß der vorliegenden Erfindung bevorzugt in Mengen von 0,01 bis 12 Gew.-%, insbesondere 0,1 bis 10 Gew.-%, vorzugsweise 0,5 bis 5 Gew.-%, bezogen auf die in Verfahrensschritt (b) resultierende Gesamtmasse, eingesetzt.

Bei der aus Verfahrensschritt (b) resultierenden Gesamtmasse handelt es sich um das Trägermaterial und die hieran bzw. hierauf aufgebrachten bzw. immobilisierten alkoholischen Extrakte sowie gegebenenfalls weitere Additive, wie beispielsweise den Granulatbildner, oder aber weitere Inhaltsstoffe, wie sie in Verfahrensschritt (b) eingesetzt werden können.

Insbesondere wird gemäß der vorliegenden Erfindung in Verfahrensschritt (b) der Träger in Mengen von 30 bis 99 Gew.-%, insbesondere 50 bis 95 Gew.-%, vorzugsweise 60 bis 90 Gew.-%, bezogen auf die in Verfahrensschritt (b) resultierende Gesamtmasse, eingesetzt.

Bevorzugt wird in Verfahrensschritt (b) der mindestens eine alkoholische Extrakt in Mengen von 0,5 bis 90 Gew.-%, insbesondere 1 bis 80 Gew.-%, vorzugsweise 5 bis 60 Gew.-%, besonders bevorzugt 10 bis 50 Gew.-%, bezogen auf die in Verfahrensschritt (b) resultierende Gesamtmasse, eingesetzt.

Was die eingesetzten Pflanzenextrakte weiterhin anbelangt, so werden in erfindungsgemäß bevorzugter Weise mindestens zwei verschiedene Pflanzenextrakte der vorgenannten Art in einer Zubereitung eingesetzt, wie beispielsweise Thymian- und Primelwurzelextrakt. Denn verschiedene Wirkstoffe unterschiedlicher Pflanzenextrakte können sich in ihrem Effekt synergistisch ergänzen bzw. verstärken, wie es z. B. bei Thymian- und Primelwurzelextrakt der Fall ist: Thymol weist nämlich neben einem leicht expektorierenden Effekt auch hervorragende antimikrobielle bzw. antivirale sowie schmerzlindernde Eigenschaften auf, wohingegen Saponine stark schleimlösend wirken. Dem Verfahrensschritt (b) schließt sich - wie zuvor geschildert - ein Verfahrensschritt (c) an, im Rahmen dessen der Alkohol entfernt wird.

Erfindungsgemäß werden in Verfahrensschritt (c) der Alkohol, insbesondere der Ethanol, und gegebenenfalls weitere flüchtige Bestandteile, insbesondere weitere Extraktionsmittel, durch Trocknung, insbesondere durch Infrarot-Trocknung, zumindest im Wesentlichen entfernt.

Dabei ist insbesondere hervorzuheben, dass die Entfernung des Alkohols sowie der eventuell weiteren flüchtigen Extraktionsmittel selektiv erfolgt. Um die Konzentration der medizinisch wirksamen Inhalts- bzw. Wirkstoffe nicht zu verringern, werden die Inhaltsstoffe bzw. Wirkstoffe des mindestens einen Pflanzenextraktes mit sekretolytischer und/oder expektorationsfördernder Wirkung durch die Trocknung zumindest weitestgehend nicht entfernt. Der maximale Trocknungsverlust beträgt insbesondere höchstens 10 Gew.-%, bevorzugterweise jedoch nicht mehr als 1,5 Gew.-%, ganz besonders bevorzugt nicht mehr als 0,1 Gew.-%, bezogen auf die Wirk- bzw. Inhaltsstoffe des mindestens einen Pflanzenextraktes.

Um dies zu ermöglichen, erfolgt die Trocknung bei Temperaturen zwischen 0 und 200 °C, insbesondere von 5 bis 150 °C, vorzugsweise jedoch im Bereich von 10 bis 100 °C, und zwar entweder bei Atmosphärendruck oder aber bei reduziertem Druck.

Dem Fachmann ist an sich bekannt, wie er die Temperatur- bzw. Druckverhältnisse an die Siedepunkte von alkoholischen Lösungs- bzw. Extraktionsmitteln anzupassen hat, um diese selektiv zu entfernen. Im Rahmen des erfindungsgemäßen Verfahrens wird die Selektivität der Entfernung nur der Lösungs- bzw. Extraktionsmittel durch die Auftragung bzw. Immobilisierung der Pflanzenextrakte an den vorgenannten Träger ermöglicht.

Erfindungsgemäß ist es bevorzugterweise vorgesehen, dass der in Verfahrenschritt (b) und/oder (c) erhaltene, den mindestens einen Pflanzenextrakt enthaltende Träger auf definierte Partikelgrößen (absolut) im Bereich von 0,01 bis 2.000 µm, insbesondere im Bereich von 0,1 bis 1.500 µm, vorzugsweise im Bereich von 1 bis 1.000 µm, besonders bevorzugt im Bereich von 5 bis 900 µm, ganz besonders bevorzugt 10 bis 800 µm, eingestellt wird.

Die Teilchengrößeneinstellung kann grundsätzlich nach Verfahrensschritt (b) und/oder (c) erfolgen; bevorzugt ist jedoch die Teilchengrößeneinstellung nach Verfahrensschritt (c).

Eine Einstellung der Partikel- bzw. Teilchengrößen erfolgt dabei insbesondere über die Granulier- bzw. Agglomerierbedingungen und ist dem Fachmann als solche bekannt. Zudem kann eine nachfolgende Einstellung bzw. Anpassung mittels Sieben oder gegebenenfalls Brechen oder Zerkleinern bzw. Klassieren erfolgen. Die Bestimmung der Partikel- bzw. Teilchengrößen erfolgt ebenfalls durch an sich bekannte Methoden, z. B. durch Siebanalyse, granulometrisch, durch Lichtbeugung oder durch mikroskopische Verfahren.

Weiterhin wird der mittels Verfahrenschritt (b) und/oder (c) erhaltene, den mindestens einen Pflanzenextrakt enthaltende Träger auf einen definierten Carr-Index von höchstens 10, insbesondere höchstens 5, vorzugsweise höchstens 1, besonders bevorzugt höchstens 0,35, ganz besonders bevorzugt höchstens 0,2, noch mehr bevorzugt höchstens 0,15, eingestellt. Der Carr-Index ist ein Indikator für die Kompressibilität eines Pulvers bzw. einer Agglomeratzusammensetzung, welcher sich über das Verhältnis der Volumendifferenz zwischen dem sich ungehindert gesetzten Pulver bzw. Granulat und dem komprimierten Pulver oder Granulat zum sich ungehindert gesetzten Volumen des Pulvers oder Granulats berechnet. In der Arzneimittelherstellung wird der Carr-Index als Indikator für die Fließfähigkeit eines Pulvers oder Granulats genutzt. Dabei gilt ein Carr-Index von mehr als 25 als Zeichen für eine äußerst schlechte Fließfähigkeit, wohingegen ein Carr-Index von weniger als 15 die gute Fließfähigkeit eines Pulvers oder Granulats aufzeigt. Insgesamt gilt somit, dass die Fließfähigkeit mit sinkendem Carr-Index besser wird. Die Einstellung des Carr-Index erfolgt im Rahmen der vorliegenden Erfindung ebenfalls über die Granulier- bzw. Agglomerierbedingungen sowie die Auswahl von Inhaltsstoffen, insbesondere des Trägermaterials und des Granulatbildners. Weiterhin werden die Fließfähigkeit bzw. der Carr-Index maßgeblich durch die Einstellung des Feuchte bzw. Trocknungsgrades der Granulate bzw. Agglomerate beeinflusst.

Weiterhin wird gemäß der vorliegenden Erfindung der in Verfahrenschritt (b) und/oder (c) erhaltene, den mindestens einen Pflanzenextrakt enthaltende Träger vorteilhafterweise auf eine definierte Dichte, insbesondere Schüttdichte, im Bereich von 0,01 und 1,0 g/ml, insbesondere im Bereich von 0,1 und 0,8 g/ml, bevorzugterweise im Bereich von 0,2 bis 0,7, eingestellt. Dies ermöglicht eine gute Dosierung und Handhabbarkeit der Agglomerate bzw. Granulate beim weiteren Einsatz zur Herstellung der finalen Zusammensetzung. Die Einstellung der definierten Schüttdichte erfolgt wie auch die des Carr-Index bzw. die der Fließfähigkeit insbesondere über die Granulier- bzw. Agglomerierbedingungen. Auch hier ist insbesondere die Wahl des Trägermaterials sowie möglicher Additive, insbesondere des Granulatbildners, von zentraler Bedeutung.

Üblicherweise umfasst der in Verfahrenschritt (c) erhaltene, den mindestens einen Pflanzenextrakt und gegebenenfalls weitere Additive enthaltende Träger den mindestens einen Pflanzenextrakt in Mengen von 0,1 bis 50 Gew.-%, insbesondere 0,2 bis 40 Gew.-%, vorzugsweise 0,5 bis 30 Gew.-%, besonders bevorzugt 0,7 bis 10 Gew.-%, bezogen auf die den mindestens einen Pflanzenextrakt, den Träger und gegebenenfalls weitere Additive enthaltende Gesamtmasse.

Im Allgemeinen entspricht der nach der Trocknung resultierende Gehalt an Pflanzenextrakt gemäß allen erfindungsgemäßen Ausführungsformen zumindest im Wesentlichen dem Gehalt an Inhalts- bzw. Wirkstoffen, wie er in der alkoholischen Ausgangszusammensetzung enthalten ist. Dies konnte von der Anmelderin experimentell nachgewiesen werden, insbesondere durch chromatographische Nachweise.

Hinsichtlich des Gehalts der Gesamtmasse an gegebenenfalls vorhandenen Additiven kann es erfindungsgemäß vorgesehen sein, dass der in Verfahrenschritt (c) erhaltene, den mindestens einen Pflanzenextrakt und gegebenenfalls weitere(s) Additiv(e) enthaltende Träger das mindestens eine weitere Additiv, insbesondere das Bindemittel und/oder den Granulatbildner, in Mengen von 0,01 bis 20 Gew.-%, insbesondere 0,1 bis 10 Gew.-%, vorzugsweise 0,5 bis 8 Gew.%, besonders bevorzugt 1 bis 5 Gew.-%, umfasst, bezogen auf die den mindestens einen Pflanzenextrakt, den Träger und gegebenenfalls weitere Additive enthaltende Gesamtmasse.

Insbesondere ist es erfindungsgemäß vorgesehen, dass die Zusammensetzung nach der vorliegenden Erfindung zumindest im Wesentlichen alkoholfrei ausgebildet ist. Insbesondere ist es zu diesem Zweck erfindungsgemäß vorgesehen, dass die Zusammensetzung nach Abschluss des Verfahrens auf einen Alkoholgehalt, insbesondere einen Ethanolgehalt, von weniger als 1,0 Vol-%, vorzugsweise weniger als 0,85 Vol.-%, besonders bevorzugt weniger als 0,5 Vol.-%, ganz besonders bevorzugt weniger als 0,25 Vol.-%, noch mehr bevorzugt weniger als 0,1 Vol.-%, am meisten bevorzugt auf etwa 0 Vol.-%, eingestellt ist. Die Einstellung des Alkoholgehalts erfolgt zum einen dadurch, dass in Verfahrensschritt (c) der Alkohol zumindest im Wesentlichen entfernt wird, und zum anderen dadurch, dass in Verfahrensschritt (d) ein zumindest im Wesentlichen alkoholfreier Exzipient zum Einsatz kommt.

Insbesondere Zusammensetzungen mit einem Alkoholgehalt, welcher geringer als 0,05 g Ethanol pro Einzeldosis bzw. geringer als 5.000 ppm oder 5 g/kg als Konzentration im finalen Produkt ist, können auch zur Anwendung bei Säuglingen, Kleinkindern und anderen Personengruppen mit Kontraindikationen bedenkenlos eingesetzt werden.

Wie zuvor beschrieben, ist die erfindungsgemäße Zusammensetzung derart beschaffen, dass sie nicht nur zumindest im Wesentlichen frei von Ethanol ist, sondern auch zumindest im Wesentlichen frei von anderen alkoholbasierten Extraktions- bzw. Lösemitteln (z. B. glykolbasierte Lösemittel, wie Glycerin, Propylenglykol etc.) ist.

Gemäß einer besonderen Ausführungsform der vorliegenden Erfindung ist es daher erfindungsgemäß bevorzugt, wenn die Zusammensetzung nach der vorliegenden Erfindung zumindest im Wesentlichen alkoholfrei ausgebildet ist bzw. zumindest im Wesentlichen frei von Alkoholen aller Art ist. Insbesondere ist es zu diesem Zweck erfindungsgemäß vorgesehen, dass die Zusammensetzung nach Abschluss des Verfahrens auf einen Gesamtalkoholgehalt, insbesondere einen Gehalt an Ethanol und gegebenenfalls weiteren Alkoholen (z. B. Glykolen etc.), von weniger als 2 Vol.-%, insbesondere weniger als 1,0 Vol-%, vorzugsweise weniger als 0,85 Vol.-%, besonders bevorzugt weniger als 0,5 Vol.-%, ganz besonders bevorzugt weniger als 0,25 Vol.-%, noch mehr bevorzugt weniger als 0,1 Vol.-%, am meisten bevorzugt auf etwa 0 Vol.-%, eingestellt ist.

Um zu gewährleisten, dass die aus Verfahrensschritt (d) resultierende finale pharmazeutische Zusammensetzung zumindest im Wesentlichen alkoholfrei ausgebildet ist und insbesondere auf einen wie zuvor spezifizierten Alkoholgehalt eingestellt wird, wird insbesondere der wässrige Exzipient in Verfahrensschritt (d) mit entsprechend geringem, vorzugsweise jedoch zumindest weitgehend ohne Alkohol bzw. vollkommen ohne Alkohol, insbesondere frei von Ethanol, ausgewählt. Wie im Folgenden noch weiter ausgeführt wird, handelt es sich bei dem wässrigen Exzipienten bevorzugterweise um eine Mischung aus Wasser sowie gegebenenfalls Saccharose und gegebenenfalls einem Sirup, welcher vorzugsweise Glucose und Fructose, bevorzugt in etwa gleichem Stoffmengenanteil (Invert-Sirup), enthält.

Zudem wird die pharmazeutische Zusammensetzung erfindungsgemäß in Verfahrensschritt (d) auf eine unter Normalbedingungen, insbesondere bei einer Temperatur von 25 °C und unter einem Druck von 1,01325 bar, flüssige und/oder fließfähige Viskosität oder Konsistenz eingestellt, um eine flüssige Dosierungsform für die orale Applikation zu gewährleisten.

Erfindungsgemäß kann es auch vorgesehen sein, dass der in Verfahrenschritt (d) eingesetzte wässrige Exzipient neben Wasser mindestens einen vorzugsweise flüssigen Zucker und/oder Zuckeraustauschstoff, bevorzugt auf Basis einer Mischung von Saccharose und einem Sirup, insbesondere einen Sirup mit Glucose und Fructose, vorzugsweise in etwa gleichem Stoffmengenanteil (Invert-Sirup), enthält. Neben der effizienten Solubilisierung der extrakthaltigen Träger verleiht ein wässriges Sirupgemisch der Zusammensetzung auch angenehme organoleptische Eigenschaften, insbesondere einen angenehm süβen Geschmack, der die starken Aromen der Inhalts- bzw. Wirkstoffe kompensiert, bzw. hierzu kompatibel ist.

Zudem kann es erfindungsgemäß vorgesehen sein, dass der wässrige Exzipient neben Wasser mindestens ein weiteres Additiv, insbesondere aus der Gruppe von Netzmitteln und/oder oberflächenaktiven Substanzen, Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen, enthält, so dass die Zusammensetzung in ihren Eigenschaften nahezu beliebig modifizierbar ist.

Insbesondere kann es erfindungsgemäß vorgesehen sein, dass der in Verfahrensschritt (d) eingesetzte wässrige Exzipient Wasser in Mengen von 5 bis 95 Gew.-%, insbesondere 10 bis 90 Gew.-%, vorzugsweise 20 bis 80 Gew.-%, bezogen auf den Exzipienten, enthält.

Weiterer erfindungsgemäßer Gegenstand - gemäß einem **zweiten** Aspekt der vorliegenden Erfindung - ist eine Zusammensetzung auf Basis mindestens eines Pflanzenextraktes mit sekretolytischer und/oder expektorationsfördernder Wirkung, insbesondere eine pharmazeutische Zubereitung, welche sich zur prophylaktischen und/oder therapeutischen Behandlung von Atemwegserkrankungen, Erkältungskrankheiten sowie grippalen Infekten und damit verbundenen Symptomen eignet und durch ein Verfahren erhältlich ist, wie es zuvor beschrieben wurde.

Insbesondere ist Gegenstand der vorliegenden Erfindung gemäß diesem Erfindungsaspekt eine Zusammensetzung, insbesondere pharmazeutische Zubereitung, zur prophylaktischen und/oder therapeutischen Behandlung von Atemwegserkrankungen, Erkältungskrankheiten sowie grippalen Infekten und damit verbundenen Symptomen, welche durch ein Verfahren wie es zuvor beschrieben wurde erhältlich ist, wobei die Zusammensetzung mindestens einen Pflanzenextrakt mit sekretolytischer und/oder expektorationsfördernder Wirkung enthält und in Form einer zumindest im Wesentlichen alkoholfreien Zubereitung mit einem Alkoholgehalt, insbesondere einem Ethanolgehalt, von weniger als 2 Vol.-% vorliegt, wobei der mindestens eine Pflanzenextrakt mit sekretolytischer und/oder expektorationsfördernder Wirkung ausgewählt ist aus der Gruppe von Thymian-Extrakt, Primelwurzel-Extrakt, Efeu-Extrakt, Pelargonie-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte.

Hinsichtlich des Begriffs "pharmazeutische Zusammensetzung" - synonym bisweilen auch als "pharmazeutische Zubereitung" bezeichnet - ist zu erwähnen, dass dieser im Rahmen der vorliegenden Erfindung sehr weit zu verstehen ist und nicht nur Arzneimittel, Medikamente, Pharmaka oder sonstige Medizinprodukte, welche laut Arzneimittelgesetz zur Diagnostik bzw. als Therapeutika bestimmt sind, bezeichnet, sondern darüber hinaus auch Nahrungsergänzungsmittel oder nicht unter das Arzneimittelgesetz fallende naturheilkundliche bzw. phytopharmazeutische Produkte, Zubereitungen oder Zusammensetzungen. Diesbezüglich kann auf obige Ausführungen verwiesen werden.

Im Rahmen der vorliegenden Erfindung wird, wie zuvor geschildert, eine zumindest im Wesentlichen alkoholfreie Zusammensetzung der vorgenannten Art bereitgestellt.

In diesem Zusammenhang kann der gegebenenfalls vorhandene Restalkoholgehalt in der erfindungsgemäßen Zusammensetzung in gewissen Grenzen variieren.

Erfindungsgemäß ist es insbesondere vorgesehen, dass die Zusammensetzung einen Alkoholgehalt, insbesondere einen Ethanolgehalt, von weniger als 1,0 Vol-%, vorzugsweise weniger als 0,85 Vol.-%, besonders bevorzugt weniger als 0,5 Vol.-%, ganz besonders bevorzugt weniger als 0,25 Vol.-%, noch mehr bevorzugt weniger als 0,1 Vol.-%, am meisten bevorzugt von etwa 0 Vol.-%, aufweist.

Insbesondere Zusammensetzungen mit einem Alkoholgehalt, welcher geringer als 0,05 g Ethanol pro Einzeldosis bzw. geringer als 5.000 ppm oder 5 g/kg als Konzentration im finalen Produkt ist, können auch zur Anwendung bei Säuglingen, Kleinkindern und anderen Personengruppen mit Kontraindikationen bedenkenlos eingesetzt werden.

Wie zuvor im Zusammenhang mit dem erfindungsgemäßen Verfahren beschrieben, ist die erfindungsgemäße Zusammensetzung derart beschaffen, dass sie nicht nur zumindest im Wesentlichen frei von Ethanol ist, sondern auch zumindest im Wesentlichen frei von anderen alkoholbasierten Extraktions- bzw. Lösemitteln (z. B. glykolbasierten Lösemittel, wie Glycerin, Propylenglykol etc.) ist.

Weiterhin ist die erfindungsgemäße Zusammensetzung bevorzugt dergestalt, dass sie unter Normalbedingungen, insbesondere bei einer Temperatur von 25 °C und unter einem Druck von 1,01325 bar, flüssig und/oder fließfähig ist.

Was den mindestens einen Pflanzenextrakt mit sekretolytischer und/oder expektorationsfördernder Wirkung in der Zusammensetzung angeht, so ist dieser aus der Gruppe von Thymian-Extrakt, Primelwurzel-Extrakt, Efeu-Extrakt, Pelargonie-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte ausgewählt.

Erfindungsgemäß bevorzugt ist eine erfindungsgemäße Zusammensetzung, welche mindestens zwei der vorgenannten Pflanzenextrakte enthält. Auf diese Weise lässt sich eine Wirkungssteigerung durch Kombinationen unterschiedlicher Wirk- bzw. Inhaltsstoffe erreichen.

Eine erfindungsgemäß besonders bevorzugte Kombination von Pflanzenextraken in der erfindungsgemäßen Zusammensetzung kann zum Beispiel mindestens einen Extrakt von Thymian und mindestens einen Extrakt von Primelwurzel in Kombination oder aber einen Thymian/Primelwurzel-Mischextrakt umfassen; dies ist mit den vorgenannten Vorteilen verbunden, insbesondere aufgrund der vorteilhaften Kombination der unterschiedlichen Inhaltsstoffe dieser beiden Drogen.

Das Droge/Extrakt-Verhältnis des mindestens einen eingesetzten Pflanzenextraktes kann grundsätzlich in weiten Bereichen variieren. Insbesondere kann es erfindungsgemäß vorgesehen sein, dass der mindestens eine Pflanzenextrakt ein Droge/Extrakt-Verhältnis im Bereich von 1 : 10 bis 10 : 1, insbesondere im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, aufweist. Wie zuvor geschildert, gewährleisten die vorgenannten Bereiche für das Droge/Extrakt-Verhältnis eine ausreichende Wirkeffizienz einerseits und eine ökonomische Verfügbarkeit andererseits.

Die Menge an Pflanzenextrakt in der erfindungsgemäßen Zusammensetzung kann in weiten Bereichen variieren. So enthält die Zusammensetzung den mindestens einen Pflanzenextrakt, berechnet als Trockenextrakt und bezogen auf die Zusammensetzung, in Mengen von 0,001 bis 50 Gew.-%, insbesondere 0,01 bis 30 Gew.-%, vorzugsweise 0,05 bis 20 Gew.-%, besonders bevorzugt 0,1 bis 10 Gew.-%. Die vorgenannten Mengen gewährleisten einerseits eine ausreichende Wirksamkeit und andererseits eine gute ökonomische Verfügbarkeit.

Wie zuvor geschildert, ist die erfindungsgemäße Zusammensetzung zumindest im Wesentlichen alkoholfrei ausgebildet.

Gemäß einer bevorzugten Ausführungsform ist die erfindungsgemäße Zusammensetzung wässrig basiert bzw. als wässrige Zubereitung formuliert bzw. umfasst einen wässrigen Exzipienten.

Die Menge an Wasser in der erfindungsgemäßen Zusammensetzung kann in weiten Bereichen variieren. Diesbezüglich kann es erfindungsgemäß vorgesehen sein, dass die Zusammensetzung Wasser, insbesondere in Mengen von 5 bis 99 Gew.-%, insbesondere 7,5 bis 70 Gew.-%, vorzugsweise 10 bis 50 Gew.-%, besonders bevorzugt 15 bis 40 Gew.-%, enthält, bezogen auf die Zusammensetzung. Die vorgenannten Wassermengen gewährleisten einerseits eine gute Formulierbarkeit der erfindungsgemäßen Zusammensetzung als flüssige bzw. fließfähige Zubereitung und andererseits eine gute orale Applizierbarkeit sowie darüber hinaus eine zumindest im Wesentlichen vollständige Solubilisierung aller Wirk- und Inhaltsstoffe.

Abgesehen von Wasser selbst kann der wässrige Exzipient aber auch noch andere Inhaltsstoffe, wie z. B. Zucker und/oder Zuckeraustauschstoffe und/oder Additive bzw. Hilfsstoffe etc., enthalten, wie nachfolgend noch im Detail geschildert.

Gemäß einer besonderen Ausführungsform kann es beispielsweise erfindungsgemäß vorgesehen sein, dass die Zusammensetzung - neben Wasser und dem mindestens einen Pflanzenextrakt - bzw. der wässrige Exzipient mindestens einen vorzugsweise flüssigen Zucker und/oder Zuckeraustauschstoff, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), aufweist. Dabei kann die erfindungsgemäße Zusammensetzung den mindestens einen Zucker und/oder Zuckeraustauschstoff insbesondere in Mengen von 5 bis 95 Gew.-%, insbesondere 10 bis 90 Gew.-%, vorzugsweise 20 bis 90 %, besonders bevorzugt 40 bis 85 Gew.-%, ganz besonders bevorzugt 60 bis 85 Gew.-%, enthalten, bezogen auf die Zusammensetzung. Hierbei sollten die Mengenverhältnisse insbesondere derart eingestellt sein, dass die erfindungsgemäße Zusammensetzung trotz des Vorhandenseins von Zucker(n) und/oder Zuckeraustauschstoff(en) oder dergleichen unter Normalbedingungen, d. h. bei einer Temperatur von 25 °C und einem Druck von 1,01325 bar, flüssig bzw. fließfähig ist, so dass eine flüssige Dosierungsform zur oralen Applikation realisiert wird.

Gemäß einer erfindungsgemäß besonders bevorzugten Ausführungsform kann die Zusammensetzung mindestens ein Additiv enthalten, insbesondere aus der Gruppe von Netzmitteln und/oder oberflächenaktiven Substanzen, Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen. Das Additiv kann dabei insbesondere in Mengen von 0,001 bis 30 Gew.-%, insbesondere 0,01 bis 20 Gew.-%, vorzugsweise 0,05 bis 10 %, besonders bevorzugt 0,1 bis 5 Gew.-%, bezogen auf die Zusammensetzung, enthalten sein.

Eine erfindungsgemäß besonders bevorzugte Zusammensetzung, insbesondere pharmazeutische Zubereitung, welche sich vorzugsweise zur prophylaktischen und/oder therapeutischen Behandlung von Atemwegserkrankungen, Erkältungskrankheiten sowie grippalen Infekten und damit verbundenen Symptomen eignet und in Form einer zumindest im Wesentlichen alkoholfreien Zubereitung vorliegt, enthält bevorzugt mindestens die folgenden Inhaltsstoffe:
- mindestens einen Pflanzenextrakt mit sekretolytischer und/oder expektorationsfördernder Wirkung, bevorzugt jeweils mit einem Droge/Extrakt-Verhältnis im Bereich von 1 : 10 bis 10 : 1, insbesondere im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, insbesondere wobei die Zusammensetzung den mindestens einen Pflanzenextrakt, berechnet als Trockenextrakt, in Mengen von 0,001 bis 50 Gew.-%, insbesondere 0,01 bis 30 Gew.-%, vorzugsweise 0,05 bis 20 Gew.-%, besonders bevorzugt 0,1 bis 10 Gew.-%, enthält und/oder insbesondere wobei der mindestens eine Pflanzenextrakt mit sekretolytischer und/oder expektorationsfördernder Wirkung ausgewählt ist aus der Gruppe von Thymian-Extrakt, Primelwurzel-Extrakt, Efeu-Extrakt, Pelargonie-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte;
- Wasser, insbesondere in Mengen von 5 bis 99 Gew.-%, insbesondere 7,5 bis 70 Gew.-%, vorzugsweise 10 bis 50 Gew.-%, besonders bevorzugt 15 bis 40 Gew.-%;
- mindestens einen vorzugsweise flüssigen Zucker und/oder Zuckeraustauschstoff, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), enthält, insbesondere wobei die Zusammensetzung den mindestens einen Zucker und/oder Zuckeraustauschstoff in Mengen von 5 bis 95 Gew.-%, insbesondere 10 bis 90 Gew.-%, vorzugsweise 20 bis 90 %, besonders bevorzugt 40 bis 85 Gew.-%, ganz besonders bevorzugt 60 bis 85 Gew.-%, enthält;
- gegebenenfalls mindestens ein Additiv, insbesondere aus der Gruppe von Netzmitteln und/oder oberflächenaktiven Substanzen, Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren; Suspensionsmitteln oder deren Mischungen, insbesondere wobei die Zusammensetzung das mindestens eine Additiv in Mengen von 0,001 bis 30 Gew.-%, insbesondere 0,01 bis 20 Gew.-%, vorzugsweise 0,05 bis 10 %, besonders bevorzugt 0,1 bis 5 Gew.-%, enthält;
wobei alle vorgenannten Gewichtsprozentangaben auf die Zusammensetzung bezogen sind.

Für weitergehende Einzelheiten zu dem zweiten Erfindungsaspekt kann zur Vermeidung unnötiger Wiederholungen auf obige Ausführungen zu dem ersten Erfindungsaspekt verwiesen werden, welche in Bezug auf den zweiten Erfindungsaspekt entsprechend gelten.

Weiterhin ist Gegenstand der vorliegenden Erfindung - gemäß einem **drit- ten** Aspekt der vorliegenden Erfindung - die Verwendung einer Zusammensetzung nach der vorliegenden Erfindung zur Herstellung eines Arzneimittels zur prophylaktischen und/oder therapeutischen Behandlung von Atemwegserkrankungen, Erkältungskrankheiten sowie grippalen Infekten und damit verbundenen Symptomen.

Für weitergehende Einzelheiten zu diesem Erfindungsaspekt kann zur Vermeidung unnötiger Wiederholungen auf obige Ausführungen zu den übrigen Erfindungsaspekten verwiesen werden, welche in Bezug auf diesen Erfindungsaspekt entsprechend gelten.

Schließlich eignet sich die erfindungsgemäße Zusammensetzung auch für ein Verfahren zur prophylaktischen und/oder therapeutischen Behandlung von Atemwegserkrankungen, Erkältungskrankheiten sowie grippalen Infekten und damit verbundenen Symptomen, wobei die erfindungsgemäße Zusammensetzung, wie sie zuvor beschrieben worden ist, in therapeutisch wirksamen Mengen verabreicht wird.

Die vorliegende Erfindung wird anhand der folgenden Ausführungsbeispiele veranschaulicht.

### Ausführungsbeispiele:

### 1. Herstellung der alkoholischen Ausgangsextrakte von Thymian und Primelwurzel

Zunächst werden separate alkoholische Extrakte von Thymian einerseits und Primelwurzel andererseits hergestellt. Die Herstellung der alkoholischen Extrakte erfolgt in an sich bekannter Weise. Als Auszugsmittel zur Herstellung des alkoholischen Thymianextraktes dient ein Lösemittelgemisch aus 10%iger Ammoniaklösung (1 Gewichtsteil), 85%igem Glycerin (20 Gewichtsteile), 90%igem Ethanol (70 Gewichtsteile) und Wasser (109 Gewichtsteile); es resultiert ein flüssiger alkoholischer Thymianextrakt mit einem Droge/Extrakt-Verhältnis von 1 : 2-2,5.

Zur Herstellung des Primelwurzelextraktes wird als Auszugsmittel 70%iges Ethanol eingesetzt; es resultiert ein flüssiger alkoholischer Primelwurzelextrakt gleichermaßen mit einem Droge/Extrakt-Verhältnis von 1: 2-2,5.

Die auf diese Weise hergestellten Extrakte werden mit einem Gewichtsverhältnis von Thymianflüssigextrakt/Primelwurzelflüssigextrakt von 2: 1 vereinigt. Nachfolgend wird ein Chromatogramm dieses Mischextraktes erstellt, um in den nachfolgenden Versuchen festzustellen, ob die Inhalts- bzw. Wirkstoffe der beiden Extrakte bei der nachfolgenden Behandlung erhalten bleiben. Des Weiteren wird der Thymolgehalt in dem Extraktgemisch bestimmt, da das Thymol nachfolgend als Indikatorsubstanz für den Nachweis einer etwaig auftretenden Veränderung des Extraktes bei der nachfolgenden Behandlung dient; Thymol, ein flüchtiges aromatisches Monoterpen und Bestandteil des Thymianextraktes, eignet sich in diesem Zusammenhang in besonderem Maße als diesbezügliche Indikatorsubstanz.

### 2. Herstellung alkoholfreier Zusammensetzungen auf Basis von Thymian- und Primelwurzelextrakten und diesbezügliche Vergleichsversuche

In einem ersten, jedoch nicht erfindungsgemäßen Ansatz wird der wie zuvor beschrieben hergestellte Mischextrakt von Thymian und Primelwurzel einer Vakuumtrocknung unterzogen, um das Extraktionsmittel, einschließlich des Alkohols, zu entfernen. Der resultierende Rückstand wird in Wasser aufgenommen und chromatographisch untersucht. Es zeigt sich, dass nicht nur ein deutlicher Substanzverlust (> 50 % ) eingetreten ist, sondern darüber hinaus charakteristische, insbesondere flüchtige Inhaltsstoffe der beiden Extrakte bei der auf diese Weise erfolgten Behandlung verloren gegangen sind. Über eine Vakuumtrocknung lässt sich ein nichtalkoholischer Extrakt bzw. eine nichtalkoholische Zusammensetzung auf Basis der beiden vorgenannten Extrakte folglich nicht erhalten. Vergleichbare Ergebnisse erhält man, wenn man andere Trocknungsmethoden anwendet, insbesondere Gefriertrocknung oder Trocknung unter erhöhten Temperaturen. Reflektiert an dem repräsentativen Inhaltsstoff Thymol aus dem Thymianextrakt, werden nach den Trocknungsversuchen jeweils nur 6,4 % bis 16,78 % des ursprünglich vorliegenden Thymols im resultierenden getrockneten Extrakt erhalten; das restliche Thymol wird im Rahmen der Trocknung entfernt.

In einem zweiten, jedoch erfindungsgemäßen Ansatz werden die vereinigten flüssigen alkoholischen Extrakte von Thymian und Primelwurzel mit einem Granulatbildner (z.B. Polyvinylpyrrolidon) versetzt und in an sich bekannter Weise in Gegenwart von Saccharose einer Wirbelschichtgranulierung unterzogen. Es resultieren mit den Extrakten von Thymian und Primelwurzel versehene partikuläre Granulate, welche nachfolgend einer Trocknung zu Zwecken der Entfernung der Extraktionsmittel und einer anschließenden Klassierung mittels Sieben unterzogen werden. Es resultieren die getrockneten Extrakte von Thymian und Primelwurzel enthaltende Granulate auf Basis von Saccharose mit Polyvinylpyrrolidon als Granulatbildner (z. B. ca. 1,5 bis 2,5 Gew.-% getrockneter Thymianextrakt, ca. 0,2 bis 1 Gew.-% getrockneter Primelwurzelextrakt, ca. 90 bis 98 Gew.-% Saccharose und ca. 2 bis 5 Gew.-% Polyvinylpyrrolidon in Abhängigkeit vom Ansatz). Die auf diese Weise erhaltenen getrockneten Granulate, welche getrockneten Thymianextrakt und getrockneten Primelwurzelextrakt enthalten, jedoch alkoholfrei sind, werden nachfolgend in einem Gemisch aus Wasser und einer Mischung aus Saccharose/Invert-Sirup aufgenommen und solubilisiert, wobei der resultierenden Mischung gegebenenfalls weitere Hilfsstoffe (z. B. Konservierungsmittel, wie z.B. Natriumbenzoat) zugesetzt werden können.

Auf diese Weise resultiert eine erfindungsgemäße, im Wesentlichen alkoholfreie Zusammensetzung auf Basis von Thymian- und Primelwurzelextrakt.

An der erfindungsgemäßen Zusammensetzung werden keine nennenswerten Alkoholmengen mehr festgestellt.

Das Chromatogramm ist gegenüber den ursprünglichen flüssigen alkoholischen Extrakten unverändert. Insbesondere ist der Thymolgehalt als Indikatorsubstanz unverändert, d. h. 100 % des ursprünglich eingesetzten Thymols werden in der resultierenden erfindungsgemäßen Zusammensetzung detektiert.

Die resultierende erfindungsgemäße Zusammensetzung kann als pharmazeutische Zubereitung für die eingangs geschilderten Indikationen verwendet werden.

Im Ergebnis stellt das erfindungsgemäße Verfahren eine effiziente wie kostengünstige Methode dar, um zumindest im Wesentlichen alkoholfreie Zusammensetzungen auf Basis von Thymian- und/oder Primelwurzelextrakten zu erhalten.

In analoger Vorgehensweise lassen sich auch erfindungsgemäße Zusammensetzungen auf Basis anderer Pflanzenextrakte der vorgenannten Art (z. B. Efeu-Extrakt, Pelargonie-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte) herstellen.

### 3. Anwendungsbeobachtungen

Die erfindungsgemäße, wie zuvor beschrieben hergestellte alkoholfreie Zusammensetzung auf Basis von Thymian- und Primelwurzelextrakten eignet sich insbesondere zur Verabreichung bei Säuglingen und Kleinkindern.

23 Probanden im Alter zwischen 7 und 38 Monaten (19 weiblich, 4 männlich), welche an Erkältungskrankheiten der oberen und unteren Atemwege mit zähflüssigem Schleim und Husten erkrankt waren, wurden mit der erfindungsgemäßen Zusammensetzung behandelt. Bei den Probanden im Alter von 6 bis 12 Monaten wurde sechsmal täglich 1,0 ml der erfindungsgemäßen Zusammensetzung über den Tag verteilt verabreicht, während bei den Probanden im Alter von mehr als 12 Monaten eine sechsmalige Verabreichung von Dosen ä 2,5 ml erfolgte.

Bereits nach drei- bis fünfmaliger Gabe der Einzeldosen trat in allen Fällen eine erhebliche Verbesserung des Allgemeinzustands auf. Insbesondere wurde ein erleichtertes Abhusten und eine verbesserte Verflüssigung des Schleims beobachtet.

Bei 18 der 23 Probanden war die Therapie nach 6 Tagen abgeschlossen, während bei den übrigen Probanden die Therapie noch weitere 6 Tage fortgeführt wurde, bis sie beendet werden konnte. In allen Fällen waren die Erkrankungen nach dieser Zeitdauer austherapiert.

Bei 6 der 23 Probanden wurden zusätzlich zu der erfindungsgemäßen Zusammensetzung ergänzende Therapeutika, insbesondere Rhinologika, verabreicht.

Weiterhin wurden 34 erwachsene Probanden im Alter von 19 bis 78 Jahren (30 männlich, 4 weiblich), welche an Erkältungskrankheiten der oberen und unteren Atemwege, verbunden mit Husten, zähflüssigem Schleim und zum Teil akuter Bronchitis, litten, mit der erfindungsgemäβen Zusammensetzung - entweder unterstützend oder als Monotherapie - behandelt. Zu diesem Zweck wurden 4- bis 6mal täglich 7,5 bis 10 ml der erfindungsgemäßen Zusammensetzung verabreicht. In allen Fällen wurde bereits nach 1 bis 2 Tagen eine erhebliche Linderung der Erkältungssymptome beobachtet. Nach 10 bis 14 Tagen waren in allen Fällen die Erkrankungen austherapiert.

Die vorstehenden Anwendungsbeobachtungen zeigen die Wirksamkeit der erfindungsgemäßen Zusammensetzung bei der Behandlung von Erkältungskrankheiten der Atemwege und den damit verbundenen Symptomen, wie Husten, zähflüssigem Schleim etc. Aufgrund der Tatsache, dass die erfindungsgemäße Zusammensetzung alkoholfrei ausgebildet ist, eignet sie sich insbesondere auch zum Einsatz bei Säuglingen und Kleinkindern. Des Weiteren wurden die organoleptischen Eigenschaften der alkoholfreien, Thymian- und Primelwurzelextrakt enthaltenden Zusammensetzung von allen Probanden als äußerst positiv bewertet.

## Patentansprüche

1. Verfahren zur Herstellung einer zumindest im Wesentlichen alkoholfreien Zusammensetzung mit einem Alkoholgehalt, insbesondere einem Ethanolgehalt, von weniger als 2 Vol.-% auf Basis mindestens eines Pflanzenextraktes mit sekretolytischer und/oder expektorationsfördernder Wirkung, wobei das Verfahren die folgenden Verfahrensschritte umfasst:
(a) Bereitstellung mindestens eines alkoholischen Pflanzenextraktes mit sekretolytischer und/oder expektorationsfördernder Wirkung, wobei der mindestens eine Pflanzenextrakt mit sekretolytischer und/oder expektorationsfördernder Wirkung ausgewählt ist aus der Gruppe von Thymian-Extrakt, Primelwurzel-Extrakt, Efeu-Extrakt, Pelargonie-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte; nachfolgend
(b) Aufbringen und/oder Immobilisieren des alkoholischen Pflanzenextraktes mit sekretolytischer und/oder expektorationsfördernder Wirkung an einem festen wasserlöslichen Träger, wobei der Träger ausgewählt wird aus der Gruppe von Zuckern, Zuckeraustauschstoffen und Kohlenhydraten sowie deren Kombinationen oder Mischungen; dann
(c) Entfernen des Alkohols durch Trocknung; anschließend
(d) Solubilisieren des auf diese Weise erhaltenen, von Alkohol befreiten, den Pflanzenextrakt mit sekretolytischer und/oder expektorationsfördernder Wirkung enthaltenden Trägers in einem wässrigen Exzipienten zum Erhalt einer im Wesentlichen alkoholfreien Zusammensetzung mit einem Alkoholgehalt, insbesondere einem Ethanolgehalt, von weniger als 2 Vol.-% auf Basis mindestens eines Pflanzenextraktes mit sekretolytischer und/oder expektorationsfördernder Wirkung.

2. Verfahren nach Anspruch 1, **dadurch gekennzeichnet,**
**dass** in Verfahrensschritt (a) der mindestens eine alkoholische Pflanzenextrakt mit einem Droge/Extrakt-Verhältnis im Bereich von 1 : 10 bis 10 : 1, insbesondere im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, bereitgestellt wird; und/oder
**dass** in Verfahrensschritt (a) der mindestens eine alkoholische Pflanzenextrakt mit einem Gehalt an Alkohol, insbesondere Ethanol, von mehr als 40 Vol.-% und/oder mehr als 40 Gew.-%, insbesondere mehr als 50 Vol.-% und/oder mehr als 50 Gew.-%, besonders bevorzugt mehr als 60 Vol.-% und/oder mehr als 60 Gew.-%, bereitgestellt wird und/oder dass in Verfahrensschritt (a) der mindestens eine alkoholische Pflanzenextrakt mit einem Gehalt an Alkohol, insbesondere Ethanol, im Bereich von 40 Vol.-% bis 99 Vol.-% und/oder im Bereich von 40 Gew.-% bis 99 Gew.-%, insbesondere im Bereich von 50 Vol.-% bis 95 Vol.% und/oder im Bereich von 50 Gew.-% bis 95 Gew.-%, besonders bevorzugt im Bereich von 60 Vol.-% bis 90 Vol.-% und/oder im Bereich von 60 Gew.-% bis 90 Gew.-%, bereitgestellt wird.

3. Verfahren nach Anspruch 1 oder 2, **dadurch gekennzeichnet, dass** in Verfahrensschritt (b) das Aufbringen und/oder Immobilisieren des mindestens einen alkoholischen Pflanzenextraktes an dem festen wasserlöslichen Träger mittels Granulierung, Agglomerierung, Pelletierung oder Coating, vorzugsweise mittels Granulierung, erfolgt, insbesondere wobei die Granulierung mittels Wirbelschichtgranulierung, Trockengranulierung oder Sprühgranulierung, vorzugsweise mittels Wirbelschichtgranulierung und/oder Sprühgranulierung, erfolgt.

4. Verfahren nach einem der vorangehenden Ansprüche, **dadurch gekennzeichnet,**
**dass** in Verfahrensschritt (b) ein partikulärer Träger eingesetzt wird und/oder dass in Verfahrensschritt (b) der Träger ausgewählt wird aus der Gruppe von Mono-, Di- Tri- und Polysacchariden, besonders bevorzugt aus Disacchariden, ganz besonders bevorzugt aus Saccharose und/oder Saccharose-Derivaten; und/oder
**dass** in Verfahrensschritt (b) das Aufbringen und/oder Immobilisieren des mindestens einen alkoholischen Pflanzenextraktes an dem Träger unter Verwendung mindestens eines weiteren Additivs, vorzugsweise mindestens eines Bindemittels und/oder Granulatbildners, insbesondere ausgewählt aus pharmazeutisch verträglichen Hilfsstoffen, besonders bevorzugt Polyvinylpyrrolidon, erfolgt, insbesondere wobei das mindestens eine weitere Additiv in Mengen von 0,01 bis 12 Gew.-%, insbesondere 0,1 bis 10 Gew.-%, vorzugsweise 0,5 bis 5 Gew.-%, bezogen auf die in Verfahrensschritt (b) resultierende Gesamtmasse, eingesetzt wird; und/oder
**dass** in Verfahrensschritt (b) der Träger in Mengen von 30 bis 99 Gew.-%, insbesondere 50 bis 95 Gew.-%, vorzugsweise 60 bis 90 Gew.-%, bezogen auf die in Verfahrensschritt (b) resultierende Gesamtmasse, eingesetzt wird; und/oder
**dass** in Verfahrensschritt (b) der mindestens eine alkoholische Pflanzenextrakt in Mengen von 0,5 bis 90 Gew.-%, insbesondere 1 bis 80 Gew.-%, vorzugsweise 5 bis 60 Gew.-%, besonders bevorzugt 10 bis 50 Gew.-%, bezogen auf die in Verfahrensschritt (b) resultierende Gesamtmasse, eingesetzt wird.

5. Verfahren nach einem der vorangehenden Ansprüche, **dadurch gekennzeichnet, dass** in Verfahrensschritt (c) der Alkohol, insbesondere Ethanol, und gegebenenfalls weitere flüchtige Bestandteile, insbesondere weitere Extraktionsmittel, durch Infrarot-Trocknung zumindest im Wesentlichen entfernt werden; und/oder dass der in Verfahrenschritt (b) und/oder (c) erhaltene, den mindestens einen Pflanzenextrakt enthaltende Träger auf definierte Partikelgrößen im Bereich von 0,01 bis 2.000 µm, insbesondere im Bereich von 0,1 bis 1.500 µm, vorzugsweise im Bereich von 1 bis 1.000 µm, besonders bevorzugt im Bereich von 5 bis 900 µm, ganz besonders bevorzugt 10 bis 800 µm, eingestellt wird; und/oder
dass der in Verfahrenschritt (b) und/oder (c) erhaltene, den mindestens einen Pflanzenextrakt enthaltende Träger auf einen definierten Carr-Index von höchstens 10, insbesondere höchstens 5, vorzugsweise höchstens 1, besonders bevorzugt höchstens 0,35, ganz besonders bevorzugt höchstens 0,2, noch mehr bevorzugt höchstens 0,15, eingestellt wird; und/oder
dass der in Verfahrenschritt (b) und/oder (c) erhaltene, den mindestens einen Pflanzenextrakt enthaltende Träger auf eine definierte Dichte, insbesondere Schüttdichte, im Bereich von 0,01 und 1,0 g/ml, insbesondere im Bereich von 0,1 und 0,8 g/ml, vorzugsweise im Bereich von 0,2 bis 0,7, eingestellt wird.

6. Verfahren nach einem der vorangehenden Ansprüche, **dadurch gekennzeichnet, dass** der in Verfahrenschritt (c) erhaltene, den mindestens einen Pflanzenextrakt und gegebenenfalls weitere Additive enthaltende Träger den mindestens einen Pflanzenextrakt in Mengen von 0,1 bis 50 Gew.-%, insbesondere 0,2 bis 40 Gew.-%, vorzugsweise 0,5 bis 30 Gew.-%, besonders bevorzugt 0,7 bis 10 Gew.-%, umfasst, bezogen auf die den mindestens einen Pflanzenextrakt, den Träger und gegebenenfalls weitere Additive enthaltende Gesamtmasse; und/oder
dass der in Verfahrenschritt (c) erhaltene, den mindestens einen Pflanzenextrakt und gegebenenfalls weitere Additive enthaltende Träger das mindestens eine weitere Additiv, insbesondere das Bindemittel und/oder den Granulatbildner, in Mengen von 0,01 bis 20 Gew.-%, insbesondere 0,1 bis 10 Gew.-%, vorzugsweise 0,5 bis 8 Gew.%, besonders bevorzugt 1 bis 5 Gew.-%, umfasst, bezogen auf die den mindestens einen Pflanzenextrakt, den Träger und gegebenenfalls weitere Additive enthaltende Gesamtmasse.

7. Verfahren nach einem der vorangehenden Ansprüche, **dadurch gekennzeichnet, dass** die Zusammensetzung auf einen Alkoholgehalt, insbesondere einen Ethanolgehalt, von weniger als 1,0 Vol.-%, vorzugsweise weniger als 0,85 Vol.-%, besonders bevorzugt weniger als 0,5 Vol.-%, ganz besonders bevorzugt weniger als 0,25 Vol.-%, noch mehr bevorzugt weniger als 0,1 Vol.-%, am meisten bevorzugt auf etwa 0 Vol.-%, eingestellt wird; und/oder
dass die Zusammensetzung in Verfahrensschritt (d) auf eine unter Normalbedingungen, insbesondere bei einer Temperatur von 25 °C und unter einem Druck von 1,01325 bar, flüssige und/oder fließfähige Viskosität oder Konsistenz eingestellt wird; und/oder
dass der in Verfahrenschritt (d) eingesetzte wässrige Exzipient neben Wasser mindestens einen vorzugsweise flüssigen Zucker und/oder Zuckeraustauschstoff, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), enthält und/oder dass der in Verfahrenschritt (d) eingesetzte wässrige Exzipient neben Wasser mindestens ein weiteres Additiv, insbesondere aus der Gruppe von Netzmitteln und/oder oberflächenaktiven Substanzen, Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen, enthält und/oder dass der in Verfahrenschritt (d) eingesetzte wässrige Exzipient Wasser in Mengen von 5 bis 95 Gew.-%, insbesondere 10 bis 90 Gew.-%, vorzugsweise 20 bis 80 Gew.-%, bezogen auf den Exzipienten, enthält.

8. Zusammensetzung auf Basis mindestens eines Pflanzenextraktes mit sekretolytischer und/oder expektorationsfördernder Wirkung, insbesondere pharmazeutische Zubereitung, zur Verwendung zur prophylaktischen und/oder therapeutischen Behandlung von Atemwegserkrankungen, Erkältungskrankheiten sowie grippalen Infekten und damit verbundenen Symptomen, erhältlich durch ein Verfahren nach einem der vorangehenden Ansprüche.

9. Zusammensetzung, insbesondere pharmazeutische Zubereitung, zur Verwendung zur prophylaktischen und/oder therapeutischen Behandlung von Atemwegserkrankungen, Erkältungskrankheiten sowie grippalen Infekten und damit verbundenen Symptomen, erhältlich durch ein Verfahren nach einem der vorangehenden Ansprüche, wobei die Zusammensetzung mindestens einen Pflanzenextrakt mit sekretolytischer und/oder expektorationsfördernder Wirkung enthält und in Form einer zumindest im Wesentlichen alkoholfreien Zubereitung mit einem Alkoholgehalt, insbesondere einem Ethanolgehalt, von weniger als 2 Vol.-% vorliegt, wobei der mindestens eine Pflanzenextrakt mit sekretolytischer und/oder expektorationsfördernder Wirkung ausgewählt ist aus der Gruppe von Thymian-Extrakt, Primelwurzel-Extrakt, Efeu-Extrakt, Pelargonie-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte.

10. Zusammensetzung zur Verwendung nach einem der vorangehenden Ansprüche 8 oder 9, **dadurch gekennzeichnet,**
**dass** die Zusammensetzung einen Alkoholgehalt, insbesondere einen Ethanolgehalt, von weniger als 1,0 Vol.-%, vorzugsweise weniger als 0,85 Vol.-%, besonders bevorzugt weniger als 0,5 Vol.-%, ganz besonders bevorzugt weniger als 0,25 Vol.-%, noch mehr bevorzugt weniger als 0,1 Vol.-%, am meisten bevorzugt von etwa 0 Vol.-%, aufweist;
und/oder
**dass** die Zusammensetzung unter Normalbedingungen, insbesondere bei einer Temperatur von 25 °C und unter einem Druck von 1,01325 bar, flüssig und/oder fließfähig ist.

11. Zusammensetzung zur Verwendung nach einem der vorangehenden Ansprüche 8 oder 9, **dadurch gekennzeichnet,**
**dass** der Pflanzenextrakt ein Droge/Extrakt-Verhältnis im Bereich von 1 : 10 bis 10 : 1, insbesondere im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, aufweist; und/oder
**dass** die Zusammensetzung den mindestens einen Pflanzenextrakt, berechnet als Trockenextrakt und bezogen auf die Zusammensetzung, in Mengen von 0,001 bis 50 Gew.-%, insbesondere 0,01 bis 30 Gew.-%, vorzugsweise 0,05 bis 20 Gew.-%, besonders bevorzugt 0,1 bis 10 Gew.-%, enthält; und/oder
**dass** die Zusammensetzung wässrig basiert ist und/oder als wässrige Zubereitung formuliert ist und/oder einen wässrigen Exzipienten umfasst; und/oder dass die Zusammensetzung Wasser enthält, insbesondere in Mengen von 5 bis 99 Gew.-%, insbesondere 7,5 bis 70 Gew.-%, vorzugsweise 10 bis 50 Gew.-%, besonders bevorzugt 15 bis 40 Gew.-%, bezogen auf die Zusammensetzung.

12. Zusammensetzung zur Verwendung nach einem der vorangehenden Ansprüche 8 oder 9, **dadurch gekennzeichnet, dass** die Zusammensetzung mindestens einen vorzugsweise flüssigen Zucker und/oder Zuckeraustauschstoff, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), enthält, insbesondere wobei die Zusammensetzung den mindestens einen Zucker und/oder Zuckeraustauschstoff in Mengen von 5 bis 95 Gew.-%, insbesondere 10 bis 90 Gew.-%, vorzugsweise 20 bis 90 Gew.-%, besonders bevorzugt 40 bis 85 Gew.-%, ganz besonders bevorzugt 60 bis 85 Gew.-%, enthält, bezogen auf die Zusammensetzung; und/oder dass die Zusammensetzung mindestens ein Additiv, insbesondere aus der Gruppe von Netzmitteln und/oder oberflächenaktiven Substanzen, Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen, enthält, insbesondere wobei die Zusammensetzung das mindestens eine Additiv in Mengen von 0,001 bis 30 Gew.-%, insbesondere 0,01 bis 20 Gew.-%, vorzugsweise 0,05 bis 10 Gew.-%, besonders bevorzugt 0,1 bis 5 Gew.-%, enthält, bezogen auf die Zusammensetzung.

13. Zusammensetzung, insbesondere pharmazeutische Zubereitung, zur Verwendung zur prophylaktischen und/oder therapeutischen Behandlung von Atemwegserkrankungen, Erkältungskrankheiten sowie grippalen Infekten und damit verbundenen Symptomen, wie in einem der vorangehenden Ansprüche 8 oder 9 definiert, wobei die Zusammensetzung in Form einer zumindest im Wesentlichen alkoholfreien Zubereitung mit einem Alkoholgehalt, insbesondere einem Ethanolgehalt, von weniger als 2 Vol.-% vorliegt und mindestens die folgenden Inhaltsstoffe enthält:
• mindestens einen Pflanzenextrakt mit sekretolytischer und/oder expektorationsfördernder Wirkung, bevorzugt jeweils mit einem Droge/Extrakt-Verhältnis im Bereich von 1 : 10 bis 10 : 1, insbesondere im Bereich von 5 : 1 bis 1 : 5, vorzugsweise im Bereich von 3 : 1 bis 1 : 4, bevorzugt im Bereich von 2 : 1 bis 1 : 3,5, ganz besonders bevorzugt im Bereich von 1 : 1 bis 1 : 3, insbesondere wobei die Zusammensetzung den mindestens einen Pflanzenextrakt, berechnet als Trockenextrakt, in Mengen von 0,001 bis 50 Gew.-%, insbesondere 0,01 bis 30 Gew.-%, vorzugsweise 0,05 bis 20 Gew.-%, besonders bevorzugt 0,1 bis 10 Gew.-%, enthält und/oder insbesondere wobei der mindestens eine Pflanzenextrakt mit sekretolytischer und/oder expektorationsfördernder Wirkung ausgewählt ist aus der Gruppe von Thymian-Extrakt, Primelwurzel-Extrakt, Efeu-Extrakt, Pelargonie-Extrakt, Holunder-Extrakt, Fenchel-Extrakt, Huflattich-Extrakt, Anis-Extrakt und Tüpfelfarn-Extrakt sowie Mischungen und Kombinationen von mindestens zwei der vorgenannten Extrakte;
• Wasser, insbesondere in Mengen von 5 bis 99 Gew.-%, insbesondere 7,5 bis 70 Gew.-%, vorzugsweise 10 bis 50 Gew.-%, besonders bevorzugt 15 bis 40 Gew.-%;
• mindestens einen vorzugsweise flüssigen Zucker und/oder Zuckeraustauschstoff, bevorzugt auf Basis einer Mischung von Saccharose und einem Sirup, insbesondere einem Sirup mit Glucose und Fructose, vorzugsweise in etwa gleichem Stoffmengenanteil (Invert-Sirup), insbesondere wobei die Zusammensetzung den mindestens einen Zucker und/oder Zuckeraustauschstoff in Mengen von 5 bis 95 Gew.-%, insbesondere 10 bis 90 Gew.-%, vorzugsweise 20 bis 90 Gew.-%, besonders bevorzugt 40 bis 85 Gew.-%, ganz besonders bevorzugt 60 bis 85 Gew.-%, enthält;
• gegebenenfalls mindestens ein Additiv, insbesondere aus der Gruppe von Netzmitteln und/oder oberflächenaktiven Substanzen, Konservierungsmitteln, Stabilisatoren, geschmacks- und/oder geruchsmodifizierenden Substanzen, Rheologiemodifikatoren, Verdickungsmitteln, Farbstoffen, Puffern, pH-Stellmitteln, Emulgatoren, Suspensionsmitteln oder deren Mischungen, insbesondere wobei die Zusammensetzung das mindestens eine Additiv in Mengen von 0,001 bis 30 Gew.-%, insbesondere 0,01 bis 20 Gew.-%, vorzugsweise 0,05 bis 10 Gew.-%, besonders bevorzugt 0,1 bis 5 Gew.-%, enthält;
wobei alle vorgenannten Gewichtsprozentangaben auf die Zusammensetzung bezogen sind.

14. Zusammensetzung, insbesondere Pharmazeutische Zubereitung, nach einem der vorangehenden Ansprüche 8 oder 9 zur Verwendung bei der prophylaktischen und/oder therapeutischen Behandlung von Atemwegserkrankungen, Erkältungskrankheiten sowie grippalen Infekten und damit verbundenen Symptomen.

15. Verwendung einer Zusammensetzung nach einem der vorangehenden Ansprüche 8 oder 9 zur Herstellung eines Arzneimittels zur prophylaktischen und/oder therapeutischen Behandlung von Atemwegserkrankungen, Erkättungskrankheiten sowie grippalen Infekten und damit verbundenen Symptomen.

## Claims

1. A method for the production of an at least essentially alcohol-free composition having an alcohol content, in particular an ethanol content, of less than 2% by volume, based on at least one plant extract having a secretolytic and/or expectoration-promoting activity, the method comprising the following process steps:
(a) providing at least one alcoholic plant extract having a secretolytic and/or expectoration-promoting activity, wherein the at least one plant extract having a secretolytic and/or expectoration-promoting activity is selected from the group of thyme extract, primrose root extract, ivy extract, geranium extract, elder extract, fennel extract, coltsfoot extract, anise extract, and polypody extract, as well as mixtures and combinations of at least two of the above mentioned extracts; subsequently
(b) applying and/or immobilizing the alcoholic plant extract, having a secretolytic and/or expectoration-promoting activity, on a solid, water soluble carrier, wherein the carrier is selected from the group of sugars, sugar substitutes, and carbohydrates, as well as the combinations or mixtures thereof; then
(c) removing the alcohol by means of drying; subsequently
(d) solubilizing the carrier obtained in this manner, which was released of alcohol, containing the plant extract having a secretolytic and/or expectoration-promoting activity, in an aqueous excipient for obtaining a composition being essentially free of alcohol, having an alcohol content, in particular ethanol content, of less than 2% by volume, based on at least one plant extract having a secretolytic and/or expectoration-promoting activity.

2. The method according to claim 1, **characterized in that**
in process step (a) the at least one alcoholic plant extract is provided at a drug/extract ratio in the range of 1:10 to 10:1, in particular in the range of 5:1 to 1:5, preferably in the range of 3:1 to 1:4, preferably in the range of 2:1 to 1:3.5, most preferred in the range of 1:1 to 1:3; and/or
in process step (a) the at least one alcoholic plant extract having a content of alcohol, in particular ethanol, of more than 40% by volume, and/or more than 40% by weight, in particular more than 50% by volume and/or more than 50% by weight, particularly preferred more than 60% by volume and/or more than 60% by weight, and/or in process step (a) the at least one alcoholic plant extract is provided with a content of alcohol, in particular ethanol, in the range of 40% by volume to 99% by volume and/or in the range of 40% by weight to 99% by weight, in particular in the range of 50% by volume to 95% by volume and/or in the range of 50% by weight to 95% by weight, particularly preferred in the range of 60% by volume to 90% by volume and/or in the range of 60% by weight to 90% by weight.

3. The method according to claims 1 or 2, **characterized in that** in process step (b) the applying and/or immobilizing of the at least one alcoholic plant extract is carried out on the solid, water soluble carrier by means of granulating, agglomerating, pelletizing, or coating, preferably by means of granulating, in particular wherein the granulating is carried out by means of fluidized bed granulating, dry granulating, or spray granulating, preferably by means of fluidized bed granulating and/or spray granulating.

4. The method according to one of the previous claims, **characterized in that** in process step (b) a particulate carrier is being utilized, and/or that in process step (b) the carrier is selected from the group of mono, di, tri, and polysaccharides, particularly preferred from disaccharides, most preferred from sucrose and/or sucrose derivatives; and/or
in process step (b) the applying and/or immobilizing of the at least one alcoholic plant extract is carried out on the carrier utilizing at least one additional additive, preferably at least one binder and/or granulator, in particular selected from pharmaceutically acceptable adjuvants, particularly preferred polyvinyl pyrrolidone, in particular wherein the at least one additional additive is utilized in amounts of 0.01 to 12% by weight, in particular 0.1 to 10% by weight, preferably 0.5 to 5% by weight, based on the total mass resulting in process step (b); and/or
in process step (b) the carrier is utilized in amounts of 30 to 99% by weight, in particular 50 to 95% by weight, preferably 60 to 90% by weight, based on the total mass resulting in process step (b); and/or
in process step (b) the at least one alcoholic plant extract is utilized in amounts of 0.5 to 90% by weight, in particular 1 to 80% by weight, preferably 5 to 60% by weight, particularly preferred 10 to 50% by weight, based on the total mass resulting in process step (b).

5. The method according to one of the previous claims, **characterized in that** in process step (c) the alcohol, in particular ethanol, and optionally additional volatile matter, in particular additional extraction agents, are removed, at least substantially, by means of infrared drying; and/or
the carrier obtained in process steps (b) and/or (c), containing at least one plant extract, is adjusted to defined particle sizes in the range of 0.01 to 2,000 µm, in particular in the range of 0.1 to 1,500 µm, preferably in the range of 1 to 1,000 µm, particularly preferred in the range of 5 to 900 µm, most preferred 10 to 800 µm; and/or
the carrier obtained in process steps (b) and/or (c), containing at least one plant extract, is adjusted to a defined Carr-index of not more than 10, in particular not more than 5, preferably not more than 1, particularly preferred not more than 0.35, more preferred not more than 0.2, most preferred not more than 0.15; and/or
the carrier obtained in process steps (b) and/or (c), containing at least one plant extract, is adjusted to a defined density, in particular bulk density, in the range of 0.01 and 1.0 g/ml, in particular in the range of 0.1 and 0.8 g/ml, preferably in the range of 0.2 to 0.7.

6. The method according to one of the previous claims, **characterized in that** the carrier obtained in process step (c), containing at least one plant extract, and optionally additional additives, comprises the at least one plant extract in amounts of 0.1 to 50% by weight, in particular 0.2 to 40% by weight, preferably 0.5 to 30% by weight, particularly preferred 0.7 to 10% by weight, based on the total mass containing the at least one plant extract, the carrier, and optionally additional additives; and/or
the carrier obtained in process step (c), containing at least one plant extract, and optionally additional additives, comprises the at least one additional additive, in particular the binder and/or the granulator, in amounts of 0.01 to 20% by weight, in particular 0.1 to 10% by weight, preferably 0.5 to 8% by weight, particularly preferred 1 to 5% by weight, based on the total mass containing the at least one plant extract, the carrier, and optionally additional additives.

7. The method according to one of the previous claims, **characterized in that** the composition is adjusted to an alcohol content, in particular an ethanol content, of less than 1.0% by volume, preferably less than 0.85% by volume, particularly preferred less than 0.5% by volume, especially preferred less than 0.25% by volume, and more preferred less than 0.1% by volume, most preferred to approximately 0% by volume; and/or
the composition is adjusted in process step (d) to a viscosity or consistency that is liquid and/or flowable under normal conditions, in particular at a temperature of 25°C and at a pressure of 1.01325 bars; and/or
in addition to water, the aqueous excipient being utilized in process step (d) also contains at least one preferably liquid sugar and/or sugar substitute, preferably based on a mixture of sucrose and a syrup, in particular a syrup having glucose and fructose, preferably at an approximately equal substance amount fraction (invert syrup), and/or that in addition of water, the aqueous excipient being utilized in process step (d) contains at least one additional additive, in particular one from the group of surfactants, and/or surface-active substances, preservatives, stabilizers, taste and/or smell modifying substances, rheology modifiers, thickeners, dyes, buffers, pH setting agents, emulsifiers, suspending agents, or the mixtures thereof, and/or that the aqueous excipient being utilized in process step (d) contains water in amounts of 5 to 95% by weight, in particular 10 to 90% by weight, preferably 20 to 80% by weight, based on the excipient.

8. A composition based on at least one plant extract, having a secretolytic and/or expectoration-promoting activity, in particular a pharmaceutical preparation for use in the prophylactic and/or therapeutic treatment of respiratory diseases, common colds, as well as influenza infections, and related symptoms, obtainable by means of a method according to one of the previous claims.

9. The composition, in particular pharmaceutical preparation for use in the prophylactic and/or therapeutic treatment of respiratory diseases, common colds, as well as influenza infections, and related symptoms, obtainable by means of a method according to one of the previous claims, wherein the composition contains at least one plant extract, having a secretolytic and/or expectoration-promoting activity, and which is present in the form of an at least essentially alcohol-free preparation having an alcohol content, in particular an ethanol content, of less than 2% by volume, wherein the at least one plant extract, having a secretolytic and/or expectoration-promoting activity, is selected from the group of thyme extract, primrose root extract, ivy extract, geranium extract, elder extract, fennel extract, coltsfoot extract, anise extract, and polypody extract, as well as mixtures and combinations of at least two of the above mentioned extracts.

10. The composition for use according to one of the previous claims 8 or 9, **characterized in that**
the composition has an alcohol content, in particular an ethanol content, of less than 1.0% by volume, preferably less than 0.85% by volume, particularly preferred less than 0.5% by volume, especially preferred less than 0.25% by volume, and more preferred less than 0.1 % by volume, most preferred to approximately 0% by volume; and/or
the composition is liquid and/or flowable under normal conditions, in particular at a temperature of 25°C and at a pressure of 1.01325 bars.

11. The composition for use according to one of the previous claims 8 or 9, **characterized in that**
the plant extract has a drug/extract ratio in the range of 1:10 to 10:1, in particular in the range of 5:1 to 1:5, preferably in the range of 3:1 to 1:4, preferably in the range of 2:1 to 1:3.5, most preferred in the range of 1:1 to 1:3; and/or
the composition contains the at least one plant extract, calculated as dry extract, and based on the composition, in amounts of 0.001 to 50% by weight, in particular 0.01 to 30% by weight, preferably 0.05 to 20% by weight, particularly preferred 0.1 to 10% by weight; and/or
the composition is based on water, and/or is formulated as an aqueous preparation, and/or comprises an aqueous excipient; and/or
the composition contains water, in particular in amounts from 5 to 99% by weight, in particular 7.5 to 70% by weight, preferably 10 to 50% by weight, particularly preferred 15 to 40% by weight, based on the composition.

12. The composition for use according to one of the previous claims 8 or 9, **characterized in that**
the composition contains at least one preferably liquid sugar and/or sugar substitute, preferably based on a mixture of sucrose and a syrup, in particular a syrup having glucose and fructose, preferably at an approximately equal substance amount fraction (invert syrup), in particular wherein the composition contains the at least one liquid sugar and/or sugar substitute in amounts of 5 to 95% by weight, in particular 10 to 90% by weight, preferably 20 to 90% by weight, particularly preferred 40 to 85% by weight, particularly preferred 60 to 85% by weight, based on the composition; and/or
the composition contains at least one additive, in particular from the group of surfactants, and/or surface-active substances, conservatives, stabilizers, taste and/or smell modifying substances, rheology modifiers, thickeners, dyes, buffers, pH setting agents, emulsifiers, suspending agents, or the mixtures thereof, in particular wherein the composition contains the at least one additive in amounts from 0.001 to 30% by weight, in particular 0.01 to 20% by weight, preferably 0.05 to 10% by weight, particularly preferred 0.1 to 5% by weight, based on the composition.

13. The composition, in particular pharmaceutical preparation, for use in the prophylactic and/or therapeutic treatment of respiratory diseases, common colds, as well as influenza infections, and related symptoms, as defined in one of the previous claims 8 or 9, wherein the composition is present in the form of an at least substantially alcohol-free preparation, having an alcohol content, in particular ethanol content, of less than 2% by volume, and contains at least the following ingredients:
• at least one plant extract, having a secretolytic and/or expectoration-promoting activity, preferably each having a drug/extract ratio in the range of 1:10 to 10:1, in particular in the range of 5:1 to 1:5, preferably in the range of 3:1 to 1:4, preferably in the range of 2:1 to 1:3.5, most preferred in the range of 1:1 to 1:3, in particular wherein the composition contains the at least one plant extract, calculated as dry extract, in amounts from 0.001 to 50% by weight, in particular 0.01 to 30% by weight, preferably 0.05 to 20% by weight, particularly preferred 0.1 to 10% by weight, and/or in particular wherein the at least one plant extract, having the secretolytic and/or expectoration-promoting activity is selected from the group of thyme extract, primrose root extract, ivy extract, geranium extract, elder extract, fennel extract, coltsfoot extract, anise extract, and polypody extract, as well as mixtures and combinations of at least two of the above mentioned extracts;
• water, in particular in amounts of 5 to 99% by weight, in particular 7.5 to 70% by weight, preferably 10 to 50% by weight, particularly preferred 15 to 40% by weight;
• at least one preferably liquid sugar and/or sugar substitute, preferably based on a mixture of sucrose and a syrup, in particular a syrup having glucose and fructose, preferably at an approximately equal substance amount fraction (invert syrup), in particular wherein the composition contains the at least one sugar and/or sugar substitute in amounts from 5 to 95% by weight, in particular 10 to 90% by weight, preferably 20 to 90% by weight, particularly preferred 40 to 85% by weight, most particularly preferred 60 to 85% by weight;
• optionally at least one additive, in particular from the group of surfactants, and/or surface-active substances, preservatives, stabilizers, taste and/or smell modifying substances, rheology modifiers, thickeners, dyes, buffers, pH setting agents, emulsifiers, suspending agents, or the mixtures thereof, in particular wherein the composition contains the at least one additive in amounts from 0.001 to 30% by weight, in particular 0.01 to 20% by weight, preferably 0.05 to 10% by weight, particularly preferred 0.1 to 5% by weight;
wherein all percentages by weight stated above are based on the composition.

14. The composition, in particular pharmaceutical preparation, according to one of the previous claims 8 or 9 for use in the prophylactic and/or therapeutic treatment of respiratory diseases, common colds, as well as influenza infections, and related symptoms.

15. A use of a composition according to one of the previous claims 8 or 9 for the production of a pharmaceutical for the prophylactic and/or therapeutic treatment of respiratory diseases, common colds, as well as influenza infections, and related symptoms.

## Revendications

1. Procédé pour la préparation d'une composition au moins pour l'essentiel sans alcool, ayant une teneur en alcool, en particulier une teneur en éthanol, inférieure à 2 % en volume sur la base d'au moins un extrait de plante ayant un effet sécrétolytique et/ou favorisant l'expectoration, le procédé comprenant les étapes suivantes :
(a) mise à disposition d'au moins un extrait alcoolique de plante ayant un effet sécrétolytique et/ou favorisant l'expectoration, le ou les extraits de plantes ayant un effet sécrétolytique et/ou favorisant l'expectoration étant choisi dans le groupe consistant en l'extrait de thym, l'extrait de racine de primevère, l'extrait de feuille de lierre, l'extrait de pélargonium, l'extrait de baie de sureau, l'extrait de fenouil, l'extrait de tussilage, l'extrait d'anis et l'extrait de polypode, ainsi que les mélanges et combinaisons d'au moins deux des extraits mentionnés ci-dessus, puis
(b) application et/ou immobilisation de l'extrait alcoolique de plante ayant un effet sécrétolytique et/ou favorisant l'expectoration sur un support solide soluble dans l'eau, le support étant choisi dans le groupe des sucres, des succédanés du sucre et les hydrates de carbone ainsi que de leurs combinaisons ou mélanges ; puis
(c) élimination de l'alcool par séchage ; puis
(d) solubilisation du support obtenu de cette manière, débarrassé de l'alcool, contenant l'extrait de plante ayant un effet sécrétolytique et/ou favorisant l'expectoration, dans un excipient aqueux pour obtenir une composition pour l'essentiel sans alcool ayant une teneur en alcool, en particulier une teneur en éthanol, inférieure à 2 % en volume sur la base d'au moins un extrait de plante ayant un effet sécrétolytique et/ou favorisant l'expectoration.

2. Procédé selon la revendication 1, **caractérisé**
**en ce que**, dans l'étape (a), le ou les extraits alcooliques de plantes sont mis à disposition avec un rapport drogue/extrait compris dans la plage de 1:10 à 10:1, en particulier dans la plage de 5:1 à 1:5, de préférence dans la plage de 3:1 à 1:4, préférentiellement dans la plage de 2:1 à 1:3,5, d'une manière tout particulièrement préférée dans la plage de 1:1 à 1:3, et/ou
**en ce que**, dans l'étape (a), le ou les extraits alcooliques de plantes sont mis à disposition avec une teneur en alcool, en particulier l'éthanol, supérieure à 40 % en volume et/ou supérieure à 40 % en poids, en particulier supérieure à 50 % en volume et/ou supérieure à 50 % en poids, d'une manière particulièrement préférée supérieure à 60 % en volume et/ou supérieure à 60 % en poids, et/ou
**en ce que**, dans l'étape (a), le ou les extraits alcooliques de plantes sont mis à disposition avec une teneur en alcool, en particulier l'éthanol, comprise dans la plage de 40 % en volume à 99 % en volume et/ou dans la plage de 40 % en poids à 99 % en poids, en particulier dans la plage de 50 % en volume à 95 % en volume et/ou dans la plage de 50 % en poids à 95 % en poids, d'une manière particulièrement préférée dans la plage de 60 % en volume à 90 % en volume et/ou dans la plage de 60 % en poids à 90 % en poids.

3. Procédé selon la revendication 1 ou 2, **caractérisé en ce que**, dans l'étape (b), l'application et/ou l'immobilisation du ou des extraits alcooliques de plantes sur le support solide soluble dans l'eau s'effectuent par granulation, agglomération, pastillage ou enrobage, de préférence par granulation, la granulation ayant lieu en particulier par granulation en lit fluidisé, granulation à sec ou granulation par pulvérisation, de préférence par granulation en lit fluidisé et/ou granulation par pulvérisation.

4. Procédé selon l'une des revendications précédentes, **caractérisé**
**en ce que**, dans l'étape (b), on utilise un support particulaire, et/ou en ce que, dans l'étape (b), le support est choisi dans le groupe consistant en les mono-, les di-, les tri- et les polysaccharides, d'une manière particulièrement préférée les disaccharides, d'une manière tout particulièrement préférée le saccharose et/ou les dérivés du saccharose ; et/ou
**en ce que**, dans l'étape (b), l'application et/ou l'immobilisation du ou des extraits alcooliques de plantes sur le support s'effectuent par utilisation d'au moins un additif supplémentaire, de préférence d'au moins un liant et/ou d'un générateur de granulés, choisi en particulier parmi les adjuvants pharmaceutiquement acceptables, d'une manière particulièrement préférée la polyvinylpyrrolidone, en particulier le ou les additifs supplémentaires étant utilisés en des quantités de 0,01 à 12 % en poids, en particulier de 0,1 à 10 % en poids, de préférence de 0,5 à 5 % en poids, par rapport à la masse totale obtenue dans l'étape (b) ; et/ou
**en ce que**, dans l'étape (b), le support est utilisé en des quantités de 30 à 99 % en poids, en particulier de 50 à 95 % en poids, de préférence de 60 à 90 % en poids, par rapport à la masse totale obtenue dans l'étape (b) ; et/ou
**en ce que**, dans l'étape (b), le ou les extraits alcooliques de plantes sont utilisés en des quantités de 0,5 à 90 % en poids, en particulier de 1 à 80 % en poids, de préférence de 5 à 60 % en poids, d'une manière particulièrement préférée de 10 à 50 % en poids, par rapport à la masse totale obtenue dans l'étape (b).

5. Procédé selon l'une des revendications précédentes, **caractérisé**
**en ce que**, dans l'étape (c), l'alcool, en particulier l'éthanol, et éventuellement d'autres constituants volatils, en particulier d'autres agents d'extraction, sont au moins pour l'essentiel éliminés par séchage par infrarouge ; et/ou
**en ce que** le support obtenu dans l'étape (b) et/ou dans l'étape (c), contenant le ou les extraits de plantes, est ajusté à des granulométries parfaitement définies comprises dans la plage de 0,01 à 2000 µm, en particulier dans la plage de 0,1 à 1500 µm, de préférence dans la plage de 1 à 1000 µm, d'une manière particulièrement préférée dans la plage de 5 à 900 µm, d'une manière tout particulièrement préférée de 10 à 800 µm ; et/ou
**en ce que** le support obtenu dans l'étape (b) et/ou dans l'étape (c), contenant le ou les extraits de plantes, est ajusté à un indice de Carr parfaitement défini, d'au plus 10, en particulier d'au plus 5, de préférence d'au plus 1, d'une manière particulièrement préférée d'au plus 0,35, d'une manière tout particulièrement préférée d'au plus 0,2, d'une manière encore plus préférée d'au plus 0,15 ; et/ou
**en ce que** le support obtenu dans l'étape (b) et/ou dans l'étape (c), contenant le ou les extraits de plantes, est ajusté à une masse volumique, en particulier une masse volumique apparente, parfaitement définie, comprise dans la plage de 0,01 à 1,0 g/ml, en particulier dans la plage de 0,1 à 0,8 g/ml, de préférence dans la plage de 0,2 à 0,7.

6. Procédé selon l'une des revendications précédentes, **caractérisé**
**en ce que** le support obtenu dans l'étape (c), contenant le ou les extraits de plantes et éventuellement des additifs supplémentaires, comprend le ou les extraits de plantes en des quantités de 0,1 à 50 % en poids, en particulier de 0,2 à 40 % en poids, de préférence de 0,5 à 30 % en poids, d'une manière particulièrement préférée de 0,7 à 10 % en poids, par rapport à la masse totale contenant le ou les extraits de plantes, le support et éventuellement des additifs supplémentaires ; et/ou
**en ce que** le support obtenu dans l'étape (c), contenant le ou les extraits de plantes et éventuellement des additifs supplémentaires, comprend le ou les additifs supplémentaires, en particulier le liant et/ou le générateur de granulés, en des quantités de 0,01 à 20 % en poids, en particulier de 0,1 à 10 % en poids, de préférence de 0,5 à 8 % en poids, d'une manière particulièrement préférée de 1 à 5 % en poids, par rapport à la masse totale contenant le ou les extraits de plantes, le support et éventuellement des additifs supplémentaires.

7. Procédé selon l'une des revendications précédentes, **caractérisé**
**en ce que** la composition est ajustée à une teneur en alcool, en particulier une teneur en éthanol, inférieure à 1,0 % en volume, de préférence inférieure à 0,85 % en volume, d'une manière particulièrement préférée inférieure à 0,5 % en volume, d'une manière tout particulièrement préférée inférieure à 0,25 % en volume, d'une manière encore plus préférée inférieure à 0,1 % en volume, et d'une manière tout spécialement préférée d'environ 0 % en volume ; et/ou
**en ce que** la composition est, dans l'étape (d), ajustée à une viscosité ou à une consistance liquide et/ou fluide dans les conditions normales, en particulier à une température de 25°C et sous une pression de 1,01325 bar ; et/ou
**en ce que** l'excipient aqueux utilisé dans l'étape (d) contient, outre de l'eau, au moins un sucre et/ou un succédané du sucre de préférence liquide, de préférence à base d'un mélange de saccharose et d'un sirop, en particulier d'un sirop avec du glucose et du fructose, de préférence selon une proportion en quantités approximativement égales (sirop inverti), et/ou que l'excipient aqueux utilisé dans l'étape (d) contient, outre de l'eau, au moins un additif supplémentaire, en particulier du groupe consistant en les mouillants et/ou les substances tensioactives, les conservateurs, les stabilisants, les substances modifiant le goût et/ou l'odeur, les agents modifiant la rhéologie, les épaississants, les substances colorantes, les tampons, les régulateurs de pH, les émulsifiants, les agents de mise en suspension ou les mélanges de ceux-ci, et/ou que l'excipient aqueux utilisé dans l'étape (d) contient de l'eau en des quantités de 5 à 95 % en poids, en particulier de 10 à 90 % en poids, de préférence de 20 à 80 % en poids, par rapport aux excipients.

8. Composition à base d'au moins un extrait de plante ayant un effet sécrétolytique et/ou favorisant l'expectoration, en particulier préparation pharmaceutique pour utilisation pour le traitement prophylactique et/ou thérapeutique de maladies des voies respiratoires, des maladies du refroidissement, ainsi que des infections grippales et des symptômes qui y sont associés, pouvant être obtenue par un procédé selon l'une des revendications précédentes.

9. Composition, en particulier préparation pharmaceutique, pour utilisation pour le traitement prophylactique et/ou thérapeutique des maladies des voies respiratoires, des maladies du refroidissement, ainsi que des infections grippales et des symptômes qui y sont associés, pouvant être obtenue par un procédé selon l'une des revendications précédentes, la composition contenant au moins un extrait de plante ayant un effet sécrétolytique et/ou favorisant l'expectoration, et se présentant sous la forme d'une préparation au moins pour l'essentiel sans alcool, ayant une teneur en alcool, en particulier une teneur en éthanol, inférieure à 2 % en volume, le ou les extraits de plantes ayant un effet sécrétolytique et/ou favorisant l'expectoration étant choisie dans le groupe consistant en l'extrait de thym, l'extrait de racine de primevère, l'extrait de feuille de lierre, l'extrait de pélargonium, l'extrait de baie de sureau, l'extrait de fenouil, l'extrait de tussilage, l'extrait d'anis et l'extrait de polypode, ainsi que les mélanges et combinaisons d'au moins deux des extraits mentionnés ci-dessus.

10. Composition pour l'utilisation selon l'une des revendications ci-dessus 8 ou 9, **caractérisée**
**en ce que** la composition présente une teneur en alcool, en particulier une teneur en éthanol, inférieure à 1,0 % en volume, de préférence inférieure à 0,85 % en volume, d'une manière particulièrement préférée inférieure à 0,5 % en volume, d'une manière tout particulièrement préférée inférieure à 0,25 % en volume, d'une manière encore plus préférée inférieure à 0,1 % en volume, d'une manière tout spécialement préférée d'environ 0 % en volume ; et/ou
**en ce que** la composition est liquide et/ou fluide dans les conditions normales, en particulier à une température de 25°C et sous une pression de 1,01325 bar.

11. Composition pour l'utilisation selon l'une des revendications précédentes 8 ou 9, **caractérisée**
**en ce que** l'extrait de plante présente un rapport drogue/extrait compris dans la plage de 1:10 à 10:1, en particulier dans la plage de 5:1 à 1:5, de préférence dans la plage de 3:1 à 1:4, de préférence dans la plage de 2:1 à 1:3,5, d'une manière tout particulièrement préférée dans la plage de 1:1 à 1:3 ; et/ou
**en ce que** la composition contient le ou les extraits de plantes, en des quantités, calculées en extrait sec et par rapport à la composition, de 0,001 à 50 % en poids, en particulier de 0,01 à 30 % en poids, de préférence de 0,05 à 20 % en poids, d'une manière particulièrement préférée de 0,1 à 10 % en poids ; et/ou
**en ce que** la composition est à base aqueuse, et/ou est formulée sous la forme d'une préparation aqueuse et/ou comprend un excipient aqueux ; et/ou
**en ce que** la composition contient de l'eau, en particulier en des quantités de 5 à 99 % en poids, en particulier de 7,5 à 70 % en poids, de préférence de 10 à 50 % en poids, d'une manière particulièrement préférée de 15 à 40 % en poids, par rapport à la composition.

12. Composition pour l'utilisation selon l'une des revendications précédentes 8 ou 9, **caractérisée**
**en ce que** la composition contient au moins un sucre et/ou un succédané du sucre de préférence liquide, de préférence à base d'un mélange de saccharose et d'un sirop, en particulier d'un sirop avec du glucose et du fructose, de préférence selon une proportion en quantités approximativement égales (sirop inverti), en particulier la composition contenant le ou les sucres et/ou succédanés du sucre en des quantités de 5 à 95 % en poids, en particulier de 10 à 90 % en poids, de préférence de 20 à 90 % en poids, d'une manière particulièrement préférée de 40 à 85 % en poids, d'une manière tout particulièrement préférée de 60 à 85 % en poids, par rapport à la composition ; et/ou
**en ce que** la composition contient au moins un additif, en particulier du groupe consistant en les mouillants et/ou les substances tensioactives, les conservateurs, les stabilisants, les substances modifiant le goût et/ou l'odeur, les agents modifiant la rhéologie, les épaississants, les substances colorantes, les tampons, les régulateurs de pH, les émulsifiants, les agents de mise en suspension ou les mélanges de ceux-ci, en particulier la composition contenant le ou les additifs en des quantités de 0,001 à 30 % en poids, en particulier de 0,01 à 20 % en poids, de préférence de 0,05 à 10 % en poids, d'une manière particulièrement préférée de 0,1 à 5 % en poids, par rapport à la composition.

13. Composition, en particulier préparation pharmaceutique, pour l'utilisation pour le traitement prophylactique et/ou thérapeutique des maladies des voies respiratoires, des maladies du refroidissement, ainsi que des infections grippales et des symptômes qui y sont associés, telle que définie dans l'une des revendications précédentes 8 ou 9, la composition se présentant sous la forme d'une préparation au moins pour l'essentiel sans alcool, ayant une teneur en alcool, en particulier une teneur en éthanol, inférieure à 2 % en volume, et contenant au moins les constituants suivantes :
• au moins un extrait de plantes ayant un effet sécrétolytique et/ou favorisant l'expectoration, de préférence chacun ayant un rapport drogue/extrait compris dans la plage de 1:10 à 10:1, en particulier dans la plage de 5:1 à 1:5, de préférence dans la plage de 3:1 à 1:4, de préférence dans la plage de 2:1 à 1:3,5, d'une manière tout particulièrement préférée dans la plage de 1:1 à 1:3, en particulier la composition contenant le ou les extraits de plantes en des quantités, calculées en extrait sec, de 0,001 à 50 % en poids, en particulier de 0,01 à 30 % en poids, de préférence de 0,05 à 20 % en poids, d'une manière particulièrement préférée de 0,1 à 10 % en poids, et/ou en particulier le ou les extraits de plantes ayant un effet sécrétolytique et/ou favorisant l'expectoration étant choisis dans le groupe consistant en l'extrait de thym, l'extrait de racine de primevère, l'extrait de feuille de lierre, l'extrait de pélargonium, l'extrait de baie de sureau, l'extrait de fenouil, l'extrait de tussilage, l'extrait d'anis et l'extrait de polypode, ainsi que les mélanges et combinaisons d'au moins deux des extraits mentionnés ci-dessus ;
• de l'eau, en particulier en des quantités de 5 à 99 % en poids, en particulier de 7,5 à 70 % en poids, de préférence de 10 à 50 % en poids, d'une manière particulièrement préférée de 15 à 40 % en poids ;
• au moins un sucre et/ou un succédané du sucre de préférence liquide, de préférence à base d'un mélange de saccharose et d'un sirop, en particulier d'un sirop avec du glucose et du fructose, de préférence selon une proportion en quantités approximativement égales (sirop inverti), en particulier la composition contenant le ou les sucres et/ou succédanés du sucre en des quantités de 5 à 95 % en poids, en particulier de 10 à 90 % en poids, de préférence de 20 à 90 % en poids, d'une manière particulièrement préférée de 40 à 85 % en poids, d'une manière tout particulièrement préférée de 60 à 85 % en poids ;
• éventuellement au moins un additif, en particulier du groupe consistant en les mouillants et/ou les substances tensioactives, les conservateurs, les stabilisants, les substances modifiant le goût et/ou l'odeur, les agents modifiant la rhéologie, les épaississants, les substances colorantes, les tampons, les régulateurs de pH, les émulsifiants, les agents de mise en suspension ou les mélanges de ceux-ci, en particulier la composition contenant le ou les additifs en des quantités de 0,001 à 30 % en poids, en particulier de 0,01 à 20 % en poids, de préférence de 0,05 à 10 % en poids, d'une manière particulièrement préférée de 0,1 à 5 % en poids,
toutes les indications de pourcentage en poids données ci-dessus étant rapportées à la composition.

14. Composition, en particulier préparation pharmaceutique, selon l'une des revendications précédentes 8 ou 9, pour utilisation lors du traitement prophylactique et/ou thérapeutique des maladies des voies respiratoires, des maladies du refroidissement, ainsi que des infections grippales et des symptômes qui y sont associés.

15. Utilisation d'une composition selon l'une des revendications précédentes 8 ou 9 pour la fabrication d'un médicament destiné au traitement prophylactique et/ou thérapeutique des maladies des voies respiratoires, des maladies du refroidissement, ainsi que des infections grippales et des symptômes qui y sont associés.
